(19) Europäisches Patentamt
European Patent Office
Office européen des brevets

(11) Numéro de publication: **0 214 879**
**B1**

# (12) FASCICULE DE BREVET EUROPEEN

(45) Date de publication du fascicule du brevet: **22.11.90**

(21) Numéro de dépôt: **86401563.1**

(22) Date de dépôt: **11.07.86**

(51) Int. Cl.$^5$: **C 08 B 37/10**, C 08 B 37/08, A 61 K 31/725, A 61 K 31/73

## (54) Procédé de sulfatation de glycosaminoglycanes, nouveaux glycosaminoglycanes obtenus et leurs applications biologiques.

(30) Priorité: **12.07.85 FR 8510787**

(43) Date de publication de la demande:
**18.03.87 Bulletin 87/12**

(45) Mention de la délivrance du brevet:
**22.11.90 Bulletin 90/47**

(84) Etats contractants désignés:
**AT BE CH DE FR GB IT LI LU NL SE**

(56) Documents cités:
**EP-A-0 148 057**
**DE-A-3 049 445**
**GB-A- 904 112**
**GB-A-2 002 406**

**HELVETICA CHIMICA ACTA, vol. 35, 1952, pages 574-588; K.H. MEYER et al.: "Les dérivés sulfatés1) de l'acide chondroîtine-sulfurique et leur action anticoagulante. Sur les polysaccharides aminés IV2)**

**Le dossier contient des informations techniques présentées postérieurement au dépôt de la demande et ne figurant pas dans le présent fascicule.**

(73) Titulaire: **SANOFI**
**40, Avenue George V**
**F-75008 Paris (FR)**

(72) Inventeur: **Petitou, Maurice**
**Tour Mexico 65, rue du Javelot**
**F-75645 Paris Cédex 13 (FR)**
Inventeur: **Choay, Jean**
**21, rue Saint-Guillaume**
**F-75007 Paris (FR)**

(74) Mandataire: **Peaucelle, Chantal et al**
**S.C. Ernest Gutmann - Yves Plasseraud 67, boulevard Haussmann**
**F-75008 Paris (FR)**

(56) References cited:
**PROCEEDINGS OF THE SOCIETY FOR EXPIRIMENTAL BIOLOGY & MEDECINE, vol. 109, 1962, pages 901-5; L. LEVY et al.: "Chemical and pharmacological studies on N-resulfated heparin"**

**DISSERTATION ABSTRACTS, vol. 21, janvier 1961, page 1747; W.W. SPENCER: "Preparation and properties of polysaccharide sulfates"**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 214 879 B1

**Description**

L'invention est relative à un procédé de sulfatation de glycosaminoglycanes.

Elle vise également de nouveaux glycosaminoglycanes, ou GAGs, de degré de sulfatation variable, et leurs applications biologiques.

Dans la description qui suit et les revendications, le terme GAG sera utilisé pour désigner indifféremment un glycosaminoglycane, une fraction, ou un fragment de glycosaminoglycane, ou encore un de leurs sels physiologiquement acceptables.

L'expression "degré de sulfatation" désigne le nombre de groupes sulfate $SO_3^-$ par unité disaccharidique acide uronique-sucre aminé (ou l'inverse) ou encore le rapport $SO_3^-/COO^-$.

On sait que les GAGs sont essentiellement formés de motifs alternés acide uronique-sucre aminé (ou l'inverse), du type de ceux rencontrés dans les chaînes oligo- et poly-saccharidiques de GAGs naturels biologiquement actifs comme l'héparine, l'héparane-sulfate, le dermatane-sulfate, les chondroïtines, les chondroïtinessulfates, ou l'acide hyaluronique.

Les motifs acide uronique répondent plus spécialement à la structure acide D-glucuronique ou L'iduronique et les motifs sucre aminé à celle de la D-glucosamine ou de la D-galactosamine.

On connaît l'importance des applications thérapeutiques des GAGs naturels ci-dessus, en particulier, pour la prévention et pour le traitement des troubles de la coagulation et des maladies liées aux désordres de la paroi vasculaire, plus spécialement des thromboses, des athéroscléroses et artérioscléroses, ou pour lutter contre le vieillissement des tissus ou des manifestations de type dégénératif telles que les alopécies.

On sait également que, par extraction, ou par dépolymérisation chimique ou enzymatique des GAGs naturels, il est possible d'obtenir des GAGs de plus faible degré de polymérisation que les produits naturels, dotés notamment d'une activité spécifique élevée vis-à-vis du facteur Xa de la coagulation sanguine. Par degré de polymérisation (ou dp en abrégé), on entent le nombre de motifs saccharidiques, acide uronique ou sucre aminé, de la chaîne glycosidique.

Des héparines dépolymérisées de degré de sulfation d'au moins 2,5, c'est-à-dire supérieur à celui des héparines naturelles et des héparines de bas poids moléculaires connues, sont décrites dans la demande EP.0116801 du 16.12.83 au nom de Anic S.p.A. et sont proposées comme médicaments à action antithrombotique hypolipémiante et fibrinolytique.

Ces héparines dépolymérisées, appelées héparines hypersulfatées dans la demande EP, sont obtenues en soumenttant une héparine naturelle, ou une de ses fractions, à l'action d'une mélange d'acide sulfurique et d'acide chlorosulfonique. Ce traitement d'hypersulfatation qui provoque une hydrolyse acide conduit à un mélange de chaînes de dp de 8 à 30.

Les travaux des inventeurs dans ce domaine les ont conduits à constater qu'en effectuant un traitement de sulfatation donné, dans certaines conditions, il est possible de modifier le degré de sulfatation de GAGs avec un rendement élevé généralement pratiquement quantitatif, sans altérer leur structure ni leur degré de polymérisation.

D'une manière avantageuse, il s'avère que la modification du taux de charge ionique des produits considérés permet d'améliorer leur spécificité d'action, notamment en augmentant certaines de leurs propriétés alors que d'autres sont diminuées.

L'invention a donc pour but de fournir un procédé de sulfatation donnant accès à des produits définis, dont le taux de charge peut être modifié comme souhaité.

Elle vise également à fournir de nouveaux GAGs à taux de charge variable, en particulier des GAGs de caractéristiques définies, notamment de degré de polymérisation homogène.

Le procédé selon l'invention de préparation de GAGs de taux de sulfatation variable est caractérisé en ce qu'on transforme un GAG donné en un sel soluble dans un solvant organique, qu'on le solubilise dans un solvant organique et qu'on traite le sel en solution par un agent de sulfatation.

Ces dispositions s'appliquent à tout GAG et permettent d'introduire, selon le degré souhaité, des groupes esters sulfuriques —$SO_3^-$ à la place des groupes —OH primaires ou secondaires sur des motifs saccharidiques du GAG de départ sand modifier son degré de polymérisation, ni son homogénéité.

Le produit obtenu ne diffère du produit de départ que par son taux de charge différent. Ses autres caractéristiques structurales sont par ailleurs connues, en particulier son degré de polymérisation.

En fonction du produit souhaité, il suffit donc d'opérer au départ le choix d'un GAG donné.

Selon un mode de réalisation de l'invention, le GAG de départ est à base des motifs alternés D-glucosamine-acide uronique, à savoir acide L'iduronique ou acide D-glucuronique. De telles alternances sont rencontrées dans l'héparine ou l'héparane-sulfate.

Dans un mode préféré de mise en oeuvre, il s'agit d'héparine, d'héparane-sulfate, de leurs fractions ou de leurs fragments.

Dans un autre mode de mise en oeuvre préféré, le GAG de départ est un GAG de bas poids moléculaire formé d'un mélange de chaînes, ou des chaînes homogènes au regard de leur degré du polymérisation, ayant un nombre de motifs sucre inférieur à celui de l'héparine ou de l'héparane-sulfate, en particulier un nombre de motifs sucre pouvant varier de 2 à 30.

Ces GAGs de bas poids moléculaire (en abrégé bas PM) comprennent, notamment, les produits suivants, à savoir:

— Les mucopolysaccharides tels qu'obtenus par extraction alcoolique, à partir d'héparine, selon le

brevet FR 2240376 du 6.11.1978 et le premier certificat d'addition 2461719 du 20.07.1979. Ces produits, selon l'un des aspects envisagés dans le brevet principal, sont formés d'un mélange de chaînes de poids moléculaire d'environ 2000 à 8000, possédant des rapports des titres Yin-Wessler/USP d'au moins 2. Parmi les produits décrits, on citera ceux possédant un rapport YW/USP de l'ordre de 3 à 5 et des poids moléculaires moyens de 3000 à 5500 tel que celui appelé ci-après CY 216.

— Les mucopolysaccharides tels qu'obtenus par dépolymérisation ménagée de l'héparine à l'aide d'acide nitreux, selon notamment le deuxième certificat d'addition 2478646 du 20.03.1980 au brevet FR 2440376 ci-dessus ou la demande de brevet FR 2503714 du 10.04.1981. Ces produits répondent d'une manière générale aux caractéristiques ci-dessus, mais sont terminés par des groupes de structure 2,5-anhydro-manno.

On citera notamment les mucopolysaccharides du type possédant une extrémité 2,5-anhydro-mannitol, tel que celui appelé ci-après CY 222, ou encore une extrémité acide 2,5-anhydro-mannonique. Les poids moléculaires des chaînes glycosidiques majoritaires sont plus spécialement de l'ordre de 2000 à 3000, notamment d'environ 2000 à 2600. Les rapports des titres Yin-Wessler d'au moins environ 200 u/mg.

— Les mucopolysaccharides essentiellement formés de chaînes (1) d'un poids moléculaire moyen de 3000 à 6000 daltons, notamment de 4000 à 5000 environ (2) possédant un titre YW/USP inférieure à 10, notamment d'environ 6 à 3, (3) terminées par des motifs de structure 2,5-anhydromanno, tels qu'obtenus par exemple selon la demande de brevet FR 2572080 du 10.10.1984.

— Les oligosaccharides formés de 8 motifs sucre au plus, de titre Yin-Wessler élevé pouvant atteindre 2000 u/mg et de titre USP faible, pratiquement nul, dotés d'un forte affinité pour l'AT—III, tels que décrits notamment dans la demande EP 0027089 du 6/10/1980.

Selon une variante, ces oligosaccharides sont obtenus par dépolymérisation nitreuse ménagée et sont terminés par un groupe de structure 2,5-anhydro-manno.

Selon une autre variante, le motif de début de chaîne correspond à un motif uronique insaturé, tel que formé en soumettant l'héparine à une dépolymérisation ménagée à l'aide d'une héparinase. En particulier, ces oligosaccharides sont formés des octosaccharides de structure ABCDEFGH répondant à la formule ci-dessous:

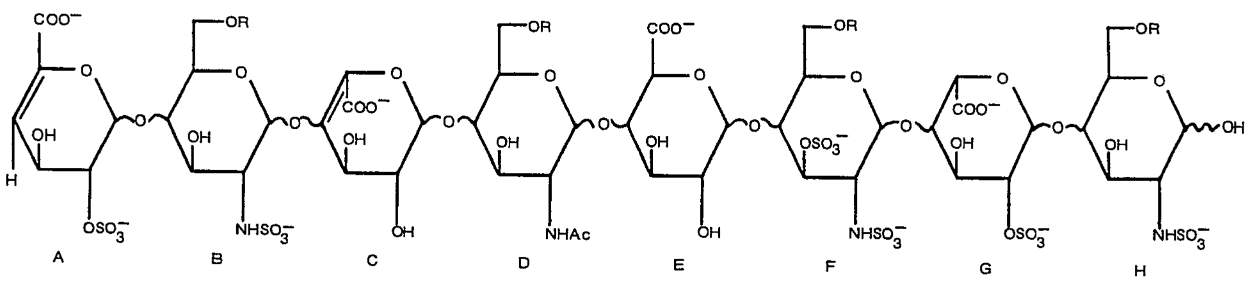

— Les compositions homogènes d'hexasaccharides, telles qu'obtenues selon la demande FR 2504928 du 29.04.1981, en soumettant les octosaccharides ci-dessus à l'action ménagée d'une héparinase. En particulier, ces compositions répondent à la formule ci-dessous:

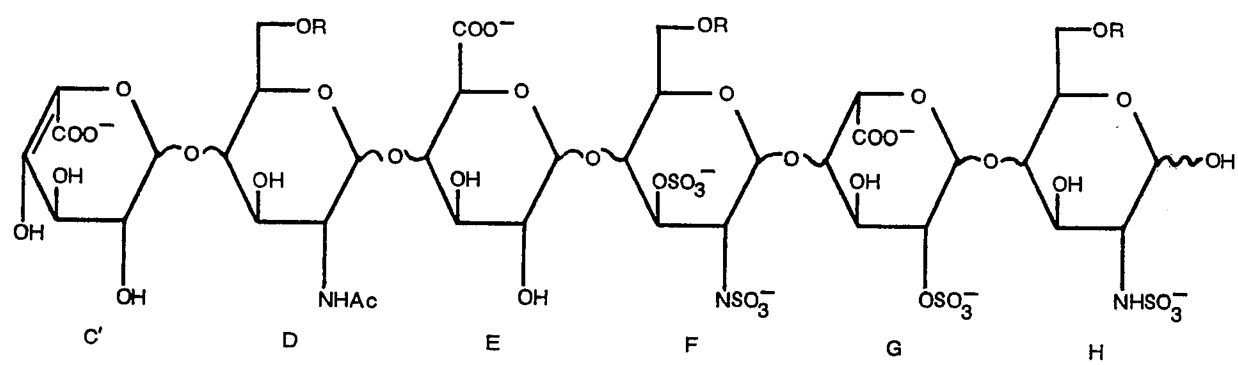

—Les oligo- ou poly-saccharides de degré de polymérisation homogène, notamment ceux obtenus par filtration, sur un matériau tel que du Sèphadex, ou par chromatographie par gradient de force ionique, des produits CY 222, CY 216, héparines, ou d'autres GAGs,

— Les GAGs tels qu'obtenus par action de periodate sur l'héparine, suivie de traitement en milieu basique, et le cas échéant d'une réduction par la borohydrure de sodium,

— Les GAGs homogènes au regard de leur degré de polymérisation, tels qu'obtenus, par exemple, par filtration sur un gel d'un mélange de dépolymérisation d'héparine,

— Les GAGs tets qu'obtenus par dépolymérisation de l'héparine avec de l'héparinase ou de l'acide nitreux, séparation des fractions possédant la séquence de fixation à l'AT—III et gel filtration de ces fractions afin d'obtenir des fragments de GAGs de degré de polymérisation varié ayant une affinité pour l'ATIII.

Il peut s'agir, par example, d'une part, de fragments de dp supérieur à 12, et d'autre part de fragments de GAGs de degré de polymérisation inférieur, notamment de dp 12, 10, 8, 6, 4 et 2 motifs sucre.

— Les GAGs tels qu'évoqués ci-dessus, mais pratiquement totalement dépourvus de la séquence capable de reconnaître spécificquement l'AT—III et de titre YW faible à nul. Il s'agit, en particulier, d'oligosaccharides qui ne se fixent par sur l'AT—III dans les procédés comportant une étape de mise en contact d'héparine ou de GAGs acec de l'AT—III, suivie de gel filtration pour obtenir des fragments homogènes de dp varié.

— Les GAGs fractions et fragments de GAGs ci-dessus possédant des enchaînements tels que rencontrés dans l'héparine, dans lesquels une partie des groupes $—NHSO_3^-$ en position 2 des motifs glucosamine, voire la majorité et même la totalité, sont remplacés par des groupes —NH-acyle, en particulier —NH-acétyle, ou par des groupes $—NH_2$.

—Les GAGs dits totaux, correspondant aux compositions de GAGs obtenues par traitement d'organes animaux.

Ces différents GAGs ne sont cités qu'à titre d'exemples, étant entendu qu'ils correspondent à des produits particulièrement bien connus de la demanderesse qui les a mis au point et décrits dans les différents brevets et demandes de brevets ci-dessus, déposés à son nom.

Mais, comme déjà indiqué, le procédé de l'invention présente l'avantage de pouvoir être mis en oeuvre avec n'importe quel type de GAG.

Ainsi, selon encore un autre mode de réalisation de l'invention, le GAG mis en oeuvre comme produit de départ est à base de motifs alternés D-galactosamineacide uronique.

Dans un mode préféré de mise en oeuvre, le GAG de départ est choisi parmi le dermatane-sulfate, les chondroïtines, les chondroïtines-sulfates ou encore l'acide hyaluronique, leurs fractions et leurs fragments.

Dans une variante, il s'agit avantageusement de dermatane-sulfate.

Selon une disposition de l'invention, un ou plusieurs groupes —OH primaires ou secondaires des motifs saccharidiques de la chaîne glycosidique des GAGs ci-dessus sont bloqués par des groupements protecteurs, ce qui permet de sulfater les autres positions comme souhaité, puis d'introduire un groupe donné à la place du groupement protecteur, puis de régénérer la fonction —OH protégée.

Ces groupes protecteurs sont choisis parmi les groupes acyle tels qu'acétyle, acétyle substitué, benzoyle.

Le sel de GAG soumis à l'opération de sulfatation est un sel soluble dans un solvant organique.

Il s'agit de préference d'un sel d'amine.

Comme sel d'amine, on a avantageusement recours à un sel d'une amine de formule $—N(R_1, R_2, R_3)$ avec $R_1$, $R_2$ et/ou $R_3$ représentant un atome d'hydrogène ou une chaîne aliphatique de 1 à 10 atomes de carbone, en particulier la triéthylamine ou la tributylamine ou encore à un sel d'ammonium quaternaire tel que le tétraéthylammonium, ou encore le tétrabutylammonium quaternaire, ou le benzéthonium.

Il est entendu que la nature de sel peut influencer la réaction de sulfatation compte tenu par exemple de l'encombrement stérique, ce qui permet de sulfater préférentiellement certaines positions.

L'obtention du sel de GAG est avantageusement effectuée en soumettant le GAG sous forme acide obtenu par utilisation d'une résine échangeuses d'ions acides, à l'action d'une amine correspondant au sel qu'on souhaite former.

La dissolution du sel de GAG est effectuée dans un solvant organique. On utilise plus spécialement un solvant aprotique dipolaire tel que le diméthyl-formamide (DMF), le diméthylsulfoxyde (DMSO), l'hexaméthylphosphotriamide (HMPT) ou l'acétonitrile. On peut également utiliser de la pyridine.

D'une mainère avantageuse, l'agent de sulfatation est choisi parmi un complexe d'anhydride sulfurique $SO_3$ avec une base organique telle que la triméthylamine (TMA) ou la pyridine, ou de l'acide chlorosulfonique dans la pyridine.

Des agents de sulfatation équivalents sont rapportés par E. E. Gilbert dans Chemical Review. 1962, vol. 62, 549—589.

L'étude des conditions de la réaction montre qu'un taux de sulfatation satisfaisant est obtenu en utilisant d'environ 1 à 5 équivalents de complexe par équivalent de OH du GAG.

La réaction de sulfatation est effectuée avantageusement à une température de l'ordre de 0°C à 100°C, notamment de l'ordre de l'ambiante ou supérieure, pendant environ 10 à 14 heures. Dans certains cas des températures inférieures à l'ambiante soit 20°C environ peuvent être utilisées.

Les conditions de réaction, notamment le solvant de réaction, la température et la durée sont choisies de manière appropriées afin d'effecturer des réactions sélectives.

Les groupes protecteurs éventuellement présents sont éliminés par réaction de saponification lorsqu'il s'agit par exemple d'un groupe de blocage O-acyle, notamment O-acétyle, ou par hydrogénation catalytique lorsqu'il s'agit par exemple d'un groupement benzoyle.

Le produit est récupéré du mélange réactionnel par une opération telle qu'une filtration sur un gel, ou

une opération d'ultrafiltration suivie d'une mise sous la forme cationique désirée à l'aide d'une résine échangeuse de cations. Le produit est ensuite précipité par un solvant miscible à l'eau et lyophilisé.

Selon une variante, pour obtenir des GAGs homogènes au regard de leur degré de polymérisation on soumet le GAG sursulfaté obtenu à une opération de gel fltration.

Les techniques dans lesquelles on utilise, entre autres, l'acide nitreux, l'héparinase ou le periodate, permettent d'obtenir des chaînes de dp pair.

Les dispositions qui précèdent permettent une sulfatation contrôlée selon le taux de sulfatation souhaité, avec un rendement élevé sur des produits définis sans dégrader leur structure osidique ni altérer leur homogénéité.

On notera également que le procédé de sulfation de l'invention qui n'ntraîne pas de modification de la structure des produits de départ, permet de disposer de GAGs à taux de sulfatation variable homogènes au regard de leur dp en effectuant une étape supplémentaire de séparation avant ou après la sulfatation.

Il est ainsi possible de préparer directement le produit qui convient pour une application donnée.

Les GAGs selon l'invention à profil de sulfatation modifié, homogènes au regard de leur degré de polymérisation ou en mélanges, sont caractérisés en ce qu'ils répondent à la formule I suivante:

$$R—(XY)_n—R'$$

dans laquelle:

R représente un acide uronique ou un acide uronique insaturé ou un groupe OH.

X représente une glucosamine ou une galactosamine dans laquelle le carbone en position 2 est substitué par un groupe $NHSO_3$—, ou dans le cas d'une glucosamine et pour des chaînes de type héparinique par un groupe $NH_2$ ou NH-acyle.

Y représente un acide uronique, à savoir un acide D-glucuronique ou L-iduronique,

n est un nombre entier de 0 à 80

R' représente une glucosamine ou une galactosamine dans laquelle le carbone en position 2 est substitué par un groupe $NHSO_3^-$, ou dans le cas de la glucosamine et pour des chaînes de type héparinique par un groupe $NH_2$, ou NH-acyle, ou encore R' représente un groupe —OH ou bien un motif glucosamine réarrangé en groupe de structure 2,5- anhydromanno ou représente un motif galactosamine réarrangé en groupe de structure 2,5-anhydrohexitol.

les groupes OH primaires ou secondaires des motifs X et Y, et, le cas échéant, de R et R', étant sulfatés de telle sorte que la position et/ou le nombre des groupes sulfate dans le chaîne de GAGs soient différents de ceux rencontrés dans les chaînes naturelles ou dépolymérisées de GAGs correspondants,

ou à leurs sels pharmacologiquement acceptables, à l'exclusion des mélanges de chaînes à extrémités non modifées ayant un nombre total de motifs glucosamineacide uronique de 8 à 30.

Une famille préférée de produits répond à la formule II:

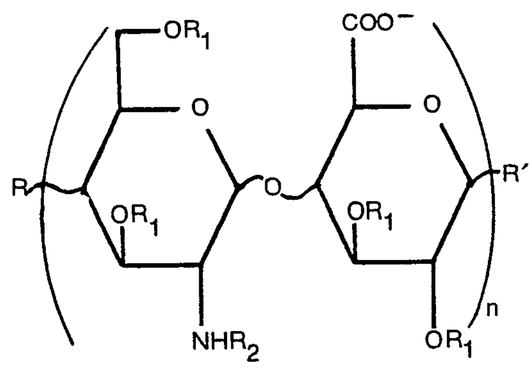

(II)

dans laquelle:

$R_1$ représente H ou $SO_3^-$

$R_2$ représente H, $SO_3^-$ ou un groupe acyle, notamment acétyle

R et R' ont les significations données ci-dessus.

Dans une autre famille préférée,

X et le cas échéant R' représentent un motif galactosamine, les liaisons entre les motif galactosamine-acide uronique étant du type 1→4β,D et celles entre les motifs acide uronique-galactosamine éventuellement présents étant du type 1→3 α, L ou 1→3 β, D.

Un groupe préféré de ces familles comprend les GAGs qui renferment un nombre de motifs sucre inférieur ou égal à 12.

Il s'agit en particulier de GAGs dans lesquels R représente un acide uronique insaturé.

En variante, il s'agit de GAGs dans lesquels R' représente un groupe de structure 2,5-anhydromanno lorsque le motif réarrangé est une glucosamine ou de structure 2,5-anhydrohexitol lorsque le motif

réarrangé est une galacotsamine. Dans des GAGs préféres, R' représente un groupe de structure 2,5-anhydromannitol.

Des GAGs avantageux de ce groupe préféré comportent un ensemble de GAGs en mélange ou homogènes au regard de degré de polymérisation dont les chaînes ont quatre, six ou huit motifs sucre.

Dans ces GAGs, R et R' représentant soit simultanément OH, soit respectivement un acide uronique et une glucosamine ou encore R' représente un groupe de structure 2,5-anhydromanno, de préférence un groupe de structure 2,5-anhydromannitol.

D'autres GAGs avantageux comportent des mélanges de chaînes de dp 8, 10 et 12 dans lesquels R et/ou R' représentent un motif sucre modifié.

D'autres GAGs avantageux comportent un ensemble homogène ou regard du degré de polymérisation de GAGs dont les chaînes ont huit, dix ou douze motifs sucre.

D'autres GAGs encore, comprennent un mélange de chaînes répondant à la formule I dans laquelle n est un nombre entier de 3 à 15, et R, et/ou R' représentent un motif sucre modifié.

Selon une variante, le carbone en position 2 de la glucosamine dans les différents groupes ci-dessus, est substitué par un groupe $NH_2$ ou NH-acyle, notamment NH-acétyle.

De tels GAGs sont préparés avantageusement à partir de produits de départ constitués de mucopolysaccharides tels qu'obtenus par dépolymerisation ménagée à l'acide nitreux, formés d'un mélange de chaînes ayant un motif terminal 2,5-anhydromanno, en particulier 2,5-anhydromannitol, de poids moléculaires de l'ordre de 2000 à 8000, possédant un rapport des titres Yin-Wessler/USP d'au moins 3, un titre Yin-Wessler d'au moins 200 u/mg et des poids moléculaires moyens de 2000 à 3000.

En variante, ces GAGs sont préparés à partir de produits de départ constitués de mucopolysaccharides tels qu'obtenus par dépolymérisation ménagée à l'acide nitreux, formés d'un mélange de chaînes ayant un motif terminal 2,5-anhydromanno, en particulier 2,5 anhydromannitol, possédant un rapport des titres Yin-Wessler/USP d'environ 3 à 6 et des poids moléculaires moyens de 4000 à 5000.

Un autre groupe préféré des familles à motifs alternés glucosamine-acide uronique ou galactosamine-acide uronique ou l'inverse, renferment un nombre de motifs sucre supérieur à 30.

D'une manière avantageuse, les GAGs obtenus selon l'invention permettent de moduler de manière spécifique une propriété biologique donnée.

Ces propriétés de modulation de l'activité biologique en fonction de la modification du profil de sulfatation ont été vérifiées sur plusieurs modèles expérimentaux.

## ACTIVITE SUR LA COAGULATION

a) activité inhibitrice vis-à-vis du facteur X activé (facteur Xa). Cette étude utilise un modèle de dosage par un substrat chromogène, le S2222 (Kabi, Stockholm), selon la méthode décrite dans Teien et Lie, 1977, Thrombos.Res., *10*, 3:399—410.

Le résultat est exprimé en unité par mg de produit testé.

b) activité inhibitrice vis-à-vis du facteur II activé (facteur IIa) ou thrombine. Le modèle consiste à étudier la concentration en glycosaminoglycane capable d'inhiber à 50% l'activité du facteur IIa en présence de l'antithombine III (AT III) ou du cofacteur II de l'héparine (HC II).

Les résultats sont exprimés en µg de produit testé par ml.

c) mesure du temps de coagulation par la méthode APTT (telle que décrite dans Caen J., Larrieu M. J. et Samama M., in l'Hemostase, Paris, Expansion Scientifique Française, 1968. pp 133—135).

Dans la cas présente, ou étudie la concentration de produit à tester qui est nécessaire pour doubler le temps de coagulation.

Le résultat est exprimé en µg de produit testé par ml.

d) activité antithrombotique in vivo chez le lapin, selon le modèle de Wessler (tel que décrit dans Wessler, S., Reimer S. M., et Sheps M. C., J. Appl. Physiol. 14 (6), 943—946, 1959).

Le produit à tester est injecté dans la carotide, à des doses variante entre 10 et 500 µg/kg, 3 mn avant l'administration de l'agent thrombogène (thromboplastine humaine).

Le résultat est exprimé en pourcentage d'inhibition de la formation du thrombus.

## ETUDE DE LA FIXATION AUX CELLULES ENDOTHELIALES

Système expérimental

— culture de cellules:

Des cultures primaires de cellules endothéliales de veine ombilicale humaine, préparées selon la technique de Jaffe et al., ont été mises en cultures dans un milieu M 199 additionné de 20% de sérum de veau foetal (SVF), en atmosphère humide contenant 5% de $CO_2$.

Entre le 5ème et le 6ème jour, on obtient des monocouches de cellules confluentes.

— expérience de liaison aux cellules endothéliales:

24 heures avant chaque essai, on enlève le milieu contenant le SVF et on lave les cultures à l'aide d'un milieu contenant 2% d'Ultroser®. l'incubation avec les héparines marquées se fait dans 2 ml de milieu. Après incubation, les cellules sont lavées 32 fois avec 1.5 ml de tampon phosphate (PBS, pH 7,4) puis détachées et solubilisées par incubation dans 0,5 ml de pronase (1 mg/ml) et 0,1% (v/v) de Triton X 100 pendant 30 mn à 37°C.

On mesure la radioactivité des cellules et du milieu d'incubation à l'aide d'un compteur gamma Beckmann 7000 ($^{125}I$).

— expérience de compétition avec l'héparine standard marquée à $^{125}I$:

Les cellules ont été incubées 5 heures avec une faible concentration d'héparine ($^{125}I$) en présence des différentes concentrations de produits froids à tester (héparine standard, héparine hypersulfatée, etc.).

La capacité à inhiber la fixation de l'héparine a été estimée par I 50 (concentration qui inhibe à 50% la fixation de l'héparine marquée). Cette valeur indique l'affinité pour les cellules endothéliales du produit testé.

ACTIVITE INHIBITRICE DU COMPLEMENT

L'héparine inhibe la génération de la $C_3$ convertase amplificatrice alterne du complément, en inhibant la formation du complexe bimoléculaire entre les protéines $C_3b$ et B.

L'effet de l'héparine et de ses dérivés sur la liaison $C_3b$-B a été étudiée en utilisant des protéines B marquées à $^{125}I$ ($^{125}I$—B) et des érythrocytes de moutons porteurs de protéine $C_3b$ ($E^SC_3b$).

On appelle IC50 la concentration d'héparine ou de l'oligosaccharide testé nécessaire pour inhiber 50% de la formation du complexe $C_3b$-B.

Cette mesure est faite in vitro, dans un système de protéines purifiées.

Système expérimental

On incube des $E^sC_3b$ ($0{,}75$—$2{,}5 \times 10^7$) dans différentes concentrations de $^{125}I$—B dans un tampon véronal contenant, 0,1% de gélatine et 5mM $Mg^{2+}$ pendant 30 mn à 30°C.

Des échantillons de 70 µl, en duplicate pour chaque réaction, sont déposés sur 300 µl d'un mélange de dibutylphtalate (Merck-Clevenot, France) et dinonylphtalate (Coger, Paris) (7, 3 v/v) dans des tubes de polypropylène de 0,5 ml.

Les tubes sont centrifugés pendant une mn à 8000 g dans un microfuge Beckman (Beckman, Paris), puis coupés juste au-dessus des précipités. La radioactivité du ligand lié est comptée.

ACTIVITE VIS-A-VIS DE L'ELASTASE ET DE LA CATHEPSINE G

Système expérimental

L'élastase leucocytaire est préparée à partir de polymorphonucléaires de rats obtenus à partir d'un exsudat pleural. Le substrate est constitué par le N-succinyl trialanine paranitroanilide et l'activité enzymatique est déterminée après incubation de l'enzyme avec le produit à tester 1 heure à température ambiante (20°C). On ajoute ensuite le substrat, on incube 20 heures à 37°C et on effectue une lecture à 410 nm.

Pour la cathepsine G humaine obtenue à partir de leucocytes polymorphonucléaires humains, le dosage est effectué sur un substrat d'azocaséine. Dans ce cas, également l'enzyme est incubée avec le produit à tester pendant 1 heure à température ambiante. On ajoute ensuite l'azocaséine et on incube le mélange pendant 5 heurs à 37°C. On précipite l'acide trichloracétique à 5% (concentration finale), on centrifuge et on effectue une lecture à 366 nm.

Cinq ensembles d'expériences ont été réalisés.

Expérience 1

ETUDE DE L'ACTIVITE ANTICOAGULANTE DE L'HEPARINE ET DU DERMATANE SULFATE SURSULFATES

a) Inhibition des Facteurs Xa et IIa en présence d'AT III ou d'HC II

| PRODUIT TESTE | Activité anti-Xa Chromogène µ/mg | Activité anti-IIa AT III IC 50 µg/ml | HC II |
|---|---|---|---|
| Héparine standard | 192 | 0,10 | 0,29 |
| Héparine sursulfatée IC 84 1545 | 79 | 0,037 | 0,045 |
| Héparine N-désulfatée N-acétylée sursulfatée IC 86 1746 | 2 | 0,38 | 0,077 |
| Dermatane sulfate | — | 100 | 5,8 |
| Dermatane sulfate sursulfaté IC 85 1610 | — | 17 | 0,34 |

On constate que l'activité anti-thrombine (anti-IIa) augmente avec le taux de sulfatation.

Dans le cas de l'héparine N-acétylé sursulfatée, l'activité via HCII est renforcée par rapport à l'héparine de départ (IC 50 passant de 0,29 à 0,077), alors que l'activité via AT III est diminuée (IC 50 passant de 0,10 à 0,38).

b) Mesure du temps de coagulation par la méthode APTT

| PRODUIT TESTE | Concentration nécessaire pour doubler le temps de coagulation — µg/ml |
|---|---|
| Héparine standard | 1,8 |
| Héparine sursulfatée IC 84 1545 | 4,5 |
| Dermatane sulfate | 50 |
| Dermatane sulfate sursulfaté IC 85 1610 | 15 |

On constate que, dans ce modèle, l'héparine sursulfatée est moins active que l'héparine normale, alors que la sursulfatation augmente l'activité du dermatane sulfate.

Des travaux complémentaires en milieu plasmatique ont montré que l'action de l'héparine normal est dépendante de l'AT III, alors que l'action de l'héparine sursulfatée est indépendante de tout cofacteur.

Ces travaux ont également montré que l'action du dermatane sulfate normale est totalement dépendante de l'HC II, alors que celle du dermatane sulfate sursulfaté ne l'est que partiellement.

c) activité antithrombotique in vivo chez le lapin du dermatane sulfate et du dermatane sulfate sursulfaté.

Les résultats obtenus dans le modèle de Wessler en utilisant comme agent thrombogène la thromboplastine humaine ont montré que la prévention de la formation du thrombus, à une dose de 100 µg/kg était de 80% pour le dermatane sulfate sursulfaté, alors qu'elle n'était que de 40% pour le dermatane sulfate à la même dose.

Expérience 2:
ETUDE DE L'ACTIVITE ANTICOAGULANTE DE FRAGMENTS D'HEPARINE DE DEGRE DE POLYMERISATION (dp) HOMOGENE SURSULFATES

Dans ce modèle, on étudie l'inhibition du Facteur IIa en mesurant l'IC 50 des produits à tester en présence d'AT III et d'HC II.

Les produits testés sont les suivants: Héparine de référence et fragments de degrés de polymérisation égaux à 4 (dp 4), 6 (db 6), 8 (dp 8), 12 (dp 12) et 16 (dp 16), ainsi que leurs homologues sursulfatés.

| PRODUIT TESTE | Activité anti-IIa | |
|---|---|---|
| | AT III | HC II<br>IC 50 µg/ml |
| Héparine standard | 0,039 | 0,14 |
| IC 84 1477 dp 4 | 100 | · |
| IC 85 1597 dp 4 sursulfaté | 100 | · |
| IC 84 1476 dp 6 | 100 | · |
| IC 85 1598 dp 6 sursulfaté | 100 | 42 |
| IC 84 1475 dp 8 | 100 | · |
| IC 85 1599 dp 8 sursulfaté | 100 | 11 |
| IC 85 1473 dp 12 | 100 | 56 |
| IC 85 1600 dp 12 sursulfaté | 100 | 3,6 |
| IC 84 1552 dp 16 | 100 | 22 |
| IC 85 1601 dp 16 sursulfaté | · | 0,68 |

· = 50% non atteints. On constate que les fragments homogènes de faible poids moléculaire avant sursulfatation n'ont pas d'activité anti-IIa vis AT III dans ce modèle, contrairement à l'héparine, puisque l'IC 50 est supérieur à 100.

La sursulfatation de ces fragments n'entraîne aucune modification sur l'activité via AT III, à l'exception du dp 16 qui, sursulfaté, acquiert une faible activité.

En revanche, l'activité anti-IIa, via HC II est accrue par la sursulfatation, en particulier lorsque le poids moléculaire augmente.

L'activité anti-IIa via Hc II reste toutefois faible par rapport à celle de l'héparine (à l'exception du fragment dp 16).

Expérience 3:
ETUDE DE LA FIXATION AUX CELLULES ENDOTHELIALES DE VEINE OMBILICALE HUMAINE, MESUREE PAR UNE EXPERIENCE DE COMPETITION AVEC DE L'HEPARINE MARQUEE ($^{125}I$).

| Substance testée | Poids moléculaire | $OSO_3^-/COO^-$ | Activité anticoagulante in vitro (μ/mg) APTT | Anti-Xa | Affinité pour les cellules endothéliales $I_{50}$(μM) |
|---|---|---|---|---|---|
| Héparine standard | 15.000 | 2.16 | 144 | 165 | 0.17 |
| Héparine sursulfatée IC 84 1545 | (6.000—25.000) | 3.01 | 60 | 35 | 0.025 |
| IC 83 1422 CY 216 | 4.500 | 2.1 | 26 | 224 | 15 |
| IC 84 1546 CY 216 sursulfaté | (1.800—3.000) | 2.5 | 22 | 30 | 0.15 |
| IC 79 797 CY 222 | 2.500 (1.500—8.00) | 2.1 | 12 | 250 | >100 |
| IC 83 1434 Fraction 8000 | 8.000 | 2.1 | 12 | 250 | 0.52 |
| IC 84 1552 dp 16 | | 2.04 | 10 | 224 | >100 |
| IC 85 1601 dp 16 sursulfaté | 5.000 | 3.35 | 15 | 23 | 0.32 |
| IC 84 1473 dp 12 | | 2.26 | 6 | 200 | >100 |
| IC 85 1600 dp 12 sursulfaté | 3.800 | 2.91 | 5 | 30 | 8.0 |
| IC 84 1475 dp 8 | | 2.41 | 3 | 143 | >100 |
| IC 85 1599 dp 8 sursulfaté | 2.500 | 2.95 | 4 | 39 | 20 |

D'une façon générale, la sursulfatation acroît la fixation aux cellules endothéliales de veine ombilicale humaine.

Cependant, les courtes chaînes n'ont que très peu d'affinité pour les cellules endothéliales.

Expérience 4:
ACTION SUR LE SYSTEME DU COMPLEMENT

Dans ce modèle, on a mesuré la capacité d'inhiber la formation de la $C_3$ convertase alterne du complément, in vitro, dans un système de protéines purifiées.

| PRODUIT TESTE | IC 50 µg/ml |
|---|---|
| Héparine standard | 0,5 |
| IC 84 1545 Héparine sursulfatée | 0,530 |
| IC 84 1444 Héparine N-désulfatée N-acétylée | 1—1,65 |
| IC 86 1746 Héparine N-désulfatée N-acétylée sursulfatée | 0,35 |
| IC 84 1552 dp 16 | 0,75 |
| IC 85 1601 dp 16 sursulfaté | 0,30 |
| IC 84 1473 dp 12 | 1,1 |
| IC 85 1600 dp 12 sursulfaté | 0,40 |
| IC 84 1475 dp 8 | 3 |
| IC 85 1599 dp 8 sursulfaté | 1,1 |
| IC 84 1476 dp 6 | 7 |
| IC 85 1598 dp 6 sursulfaté | 1,75 |
| IC 84 1477 dp 4 | 30 |
| IC 85 1597 dp 4 sursulfaté | 7 |

Les résultats montrent que la sursulfatation ne modifie pas l'activité de l'héparine.

En revanche, l'activité inhibitrice de l'heparine N-désulfatée N-acétylée et celle des fragments de dp 4 à 16 est augmentée par la sursulfatation.

Expérience 5:
ACTIVITE INHIBITRICE VIS-A-VIS DE L'ELASTASE ET DE LA CATHEPSINE G
DE FRAGMENTS DE BAS POIDS MOLECULAIRE ISSUS DU CY 222

| PRODUIT TESTE | % d'INHIBITION | |
|---|---|---|
| | Elastase | Cathepsine G |
| Héparine standard | 100% | — |
| IC 84 1545 Héparine sursulfatée | 100% | — |
| IC 84 1555 dp 22 | 91% | 53% |
| IC 84 1554 dp 20 | 92% | 54% |
| IC 84 1552 dp 16 | 82% | 49% |
| IC 85 1601 dp 16 sursulfaté | 100% | 60% |
| IC 84 1473 dp 12 | 92% | 46% |
| IC 85 1600 dp 12 sursulfaté | 97% | 51% |
| IC 84 1475 dp 8 | 53% | 34% |
| IC 85 1599 dp 8 sursulfaté | 95% | 53% |
| IC 84 1476 dp 6 | 38% | 8% |
| IC 85 1598 dp 6 sursulfaté | 91% | 43% |
| IC 84 1477 dp 4 | 28% | 7% |
| IC 85 1597 dp 4 sursulfaté | 93% | |

On constate que, pour certaine tailles moléculaires la capacité inhibitrice est fortement accrue par la sursulfatation.

Ce phénomène est particulièrement visible pour les plus petits poids moléculaires (dp 4, dp 6).

En conclusion, on voit que, selon les modèles expérimentaux (action sur la coagulation ou action hors le système de la coagulation), la sursulfatation n'a pas les mêmes répercussions.

On constante en particulier que pour les fragments de très bas poids moléculaire (4 à 8 motifs), l'activité inhibitrice vis-à-vis du système du complément et vis-à-vis de l'élastase est fortement accrue par la sursulfatation, alors que l'action sur la coagulation n'est que peu ou pas modifiée, et reste très faible.

L'effet in vivo des produits de l'invention a été testé à l'aide des deux modèles expérimentaux suivants:

— un modèle dans lequel on a utilisé des rats sensibilisés au dextran en opérant selon le protocole ci-dessous:

Des rats sont sensiblisés au dextran par injection de 10 à 50 mg de dextran en suspension dans de l'adjuvant incomplet de Freund.

La sensibilisation est effectuée par 3 séries d'injection sous-cutanées de dextran, la dose à administrer étant répartie en 10 injections à chaque fois faites à différents points du corps de l'animal, les séries d'injection sont faites à 3 semaines d'intervalle.

Les animaux sensibilisés sont répartis en 2 groupes recevant soit du dextran seul, soit du dextran plus les produits à tester. Un groupe d'animaux ne recevant rien, sert de témoin négatif.

Les produits à tester sont injectés soit par voie intraveineuse 1/4 heure avant l'injection du dextran, soit par voie sous-cutanée 1 à 2 H avant l'injection du dextran, à une dose allant de 1 à 10 mg par kilo d'animal.

Les résultats sont évalués sur un échantillon du plasma des animaux traités (0,5 ml) prélevé à 5 mn, 30 mn et 4 H. après l'injection.

Les critères des résultats sont les suivants:

a) Mesure de la lyse d'érythrocytes de mouton recouverts d'anti-corps de lapin anti-érythrocytes de mouton (test CH 50).

Des dilutions successives sont testées et les résultats sont exprimés en pourcent du plasma de la lyse des érythrocytes à chacun des dilutions, la lyse est appréciée par la quantité d'hémoglobine libérée, la mesure étant faite par densité optique. (Kabat E. A., et Meyer, in Experimental Immunochemiscry, 2ème édition, éditeur Charles C. Thomas, Springfield, Ill, USA. (1967), p. 149 — Kazatchkine N. D. Hauptmann G., et Nydegger U., in Technique du Complement, édition INSERM, Paris, (1985) p 22—30).

b) Test d'hémaglutination

Ce test utilise des érythrocytes de mouton recouverts de dextran et on mesure dans des plaques de microtitration la précipitation de ces érythrocytes avec les anti-corps anti-dextran présents dans le plasma de l'animal, les résultats étant exprimés par la plus grande dilution du plasma permettant la précipitation.

— un modèle expérimental dans lequel on utilise l'arthrite provoquée chez le rat par l'administration de l'adjuvant complet de Freund, en opérant selon le protocole ci-dessous:

Des rats mâles Lewis (160—200 g) reçoivent une injection d'adjuvant complet de Freund (FCA 0,1 ml de mycobactérium Butyricum et une suspension d'huile de paraffine 6 mg/ml), l'injection est effectuée dans la voûte plantaire de la patte postérieure gauche.

3 groupes d'animaux sont utilisés. L'un reçoit uniquement FCA, l'autre FCA plus le produit à tester, un groupe d'animaux ne reçoit aucun traitement et sert de témoin.

Les produits à tester sont administrés par voie intraveineuse ou sous-cutanée à ces taux allant de 1 à 10 mg/kg par jour pendant les 19 premiers jours suivant l'injection de FCA.

Les résultats sont appréciés d'une part par la mesure du volume de la patte arrière droite non injectée (lésion secondaire), et d'autre part par la détermination de l'index arthritique. (Perper R. J., Alvarez B., Colombo C., Fchroder H., (1971). Proc. Soc. Exp. Biol. Med., *137*, p. 506—512 — Bartlett R. R., Schleyerbach R., (1985), Int. J. Immunopharmac. Vol. 7, N° 1, p. 7—18).

Une première étude de l'octosaccharide sursulfaté de l'exemple 5 a montré un effet favorable sur ces deux modèles.

Les GAGs à taux de charge variable de l'invention sont, en outre, avantageusement dépourvus de toxicité.

La DL 50 a été étudiée par voie intraveineuse sur des souris "swiss CF" femelles, d'un poids de 20 g réparties par lot de cinq animaux. Volume d'injection: 0,5 ml, posologie finale 62, 5 mg/kg, 125 mg/kg, 250 mg/kg, 500 mg/kg, 1000 mg/kg.

Aucun animal n'est décédé au cours de l'injection ni pendant les 7 jours d'observations qui ont suivis.

Ces produits sont donc particulièrement précieux pour l'élaboration de médicaments.

Les produits de dp supérieur à 12, qui ont conservé une activité anticoagulante et antithrombotique, sont utilisables pour la prévention et le traitement des thromboses. Les produits de dp inférieur, en particulier ceux de dp 8 ou inférieur sont particulièrement précieux pour le traitement des troubles de la paroi vasculaire, du vieillissement des tissus et des manifestations de type dégénératif, notamment des troubles dus à certaines formes de déséquilibres immunitaires comme les glomérulonéphrites, les arthrites rhumatoïdes et certaines manifestations d'hypersensibilités retardées pouvant se traduire par des manifestations allergiques.

Ils peuvent également être utilisés dans des états de chocs, notamment dans des brûlures graves.

Les préparations pharmaceutiques selon l'invention sont caractérisées en ce qu'elles renferment une quantité afficace des GAGs définis ci-dessus en association avec des excipients pharmaceutiques. D'une manière avantageuse, ces préparations pharmaceutiques sont dépourvues de substances pyrogènes lorsqu'elles sont destinées à l'administration parenténale.

Des compositions préférées comprennent un véhicule pharmaceutique approprié pour l'administration par voie orale. Des formes d'administration de l'invention correspondantes comprennent avantageusement des gélules gastrorésistances, des comprimés ou tablettes, des pilules, ou encore se présentent sous forme de liposomes.

D'autres compositions pharmaceutiques comprennent ces GAGs en association avec les excipients appropriés pour l'administration par voie rectale. Des formes d'administration correspondantes sont constituées par des suppositoires.

Dans d'autres compositions pharmaceutiques, les GAGs se présentent sous forme d'aérosols ou de pommades.

L'invention concerne également des compositions pharmaceutiques injectables, stériles ou stérilisables pour l'administration tant par voie intraveineuse qu'intramusculaire ou sous-cutanée.

Ces solutions renferment avantageusement de 1 à 200 mg/ml de GAGs, de préférence de 20 à 150 mg/ml lorsque ces solutions sont destinées à l'injection par voie sous-cutanée. Elles peuvent contenir, par exemple, de notamment 30 à 100 mg/ml, notamment de 40 à 50 mg/ml de GAGs lorsqu'elles sont destinées à l'injection par voie intraveineuse ou par perfusion.

Avantageusement, de telles préparations pharmaceutiques sont présentées sous la forme de seringues non récupérables, prêtes à l'emploi.

Les compositions pharmaceutiques de l'invention sont particulièrement adaptées pour le contrôle (préventif ou curatif) de certaines étapes de la coagulation du sang chez l'homme ou l'animal, notamment dans le cas où le patient est soumis à des risques d'hypercoagulabilité résultant notamment d'opérations chirurgicales, de processus athéromateux, de développement de tumeurs et de troubles de la coagulation par des activateurs bactériens ou enzymatiques.

Certaines compositions sont capables de moduler l'action du complément, notamment, dans les syndromes de type inflammatoire, tels que ceux impliqués dans les arthrites rhumathoïdes. On sait en effet que certains des troubles observés peuvent être dus, du moins en partie, à la présence en grande quantité de complexes antigène-anticorps au niveau de l'articulation dans lequel le complément joue un rôle.

D'autres compositions sont efficaces pour lutter d'une manière générale, contre le vieillissement des tissus ou des manifestations de type dégénératif telles que les alopécies.

Afin d'illustrer l'invention, on indique, ci-après, un exemple de posologie utilisable chez l'homme: cette posologie comprend l'administration d'environ 1 mg à 1 g/24 heures, de préférence d'environ 5 à 500 mg/24 heures, par exemple de l'ordre de 200 mg/24 heures par voie intraveineuse, en administrations discontinues ou à intervalles réguliers ou de l'ordre de 200 à 1000 mg/24 heures par voie orale. Ces doses peuvent être naturellement ajustées pour chaque patient en fonction des résultats et des analyses de sang effectuées auparavant, la nature des affections dont il souffre et, d'une manière générale, de son état de santé.

L'invention concerne également encore l'application des GAGs selon l'invention à la constitution de réactifs biologiques utilisables au laboratoire, notamment, à titre de référence de comparaison pour l'étude d'autres produits dont est testée l'activité inhibitrice de protéases.

D'autres caractéristiques et avantages de l'invention apparaîtront dans les exemples qui suivent relatifs à la synthèse de produits selon l'invention et en se reportant aux figures 1 à 5.

— les figures 1 et 2 représentant respectivement les spectres de RMN de l'héparine et d'une héparine sulfatée selon l'invention,

— la figure 3, le spectre de RMN d'une héparine de bas poids moléculaire sulfatée selon l'invention

— les figures 4 et 5, les spectres de RMN respectivement d'une héparine N-acétylée et du produit correspondant sulfaté selon l'invention, (référence interne acide 3-triméthylsilyl propionique ou TSP dans l'eau lourde $^{13}C$).

## Exemple 1

Préparation d'héparine à profil de sulfatation modifié (IC 841545)

On prépare tout d'abord l'héparinate de triéthylammonium (TEA), puis on soumet ce produit à un traitement de sulfatation.

a. Préparation de l'héparinate de triéthylammonium (TEA).

1 g d'héparinate de sodium dissous dans 50 ml d'eau est converti en acide héparinique au moyen d'une résine échangeuse d'ions (Dowex® 50, $H^+$). Après neutralisation par la triéthylamine et concentration à sec, on obtient 1,3 g de sel de TEA.

b. Sulfatation

A 0,97 g du sel ci-dessus, dissous 20 ml de diméthylformamide (DMF), on ajoute le complexe sulfatant $TMA/SO_3$ (6,8 g) Après 24 heures à 50°C on dilue le mélange réactionnel, puis on récupère le produit par passage sur une colonne de gel Sephadex® G—25. Le sel de sodium est obtenu par neutralisation de l'acide issu d'un échange sur résine Dowex® 50 $H^+$. Après lyophilisation, on récupère 774 mg de produit.

c. Caractérisation

L'héparine obtenue possède un degré de sulfatation (sulfate/carboxylate; $SO_3^-/COO^-$) de 3,01 contre 2,16 pour le produit de départ.

En limitant le temps de réaction à une heure, on obtient: $SO_3^-/COO^- = 2,70$.

En présence de 0,68 g de complexe sulfatant et avec un traitement d'une heure, on obtient: $SO_3^-/COO^- = 2,40$.

Les spectres RMN de l'héparine et de l'héparine sursulfatée sont rapportés sur les figures 1 et 2. On constate en particulier la disparition du signal $CH_2OH$ à 62,4 ppm due à la transformation en $CH_2O\ SO_3$. Des modifications importantes sont également observées dans la région des carbones anomères (environ 100 ppm).

Exemple 1 bis

Variante de préparation d'une héparine à profil de sulfatation modifié

On opère comme dans l'exemple 1 mais en utilisant comme agent de sulfatation de l'acide chlorosulfonique dans la pyridine à la place du complexe $TMA/SO_3$. Dans la sulfatation, on utilise 0,97 g du sel de TEA, dissous 50 ml de pyridine, puis on ajoute l'acide chlorosulfonique (1 g.).

Après 24 h à 0°C, le mélange réactionnel est dilué et traité comme indiqué dans l'exemple 1.

On obtient une héparine présentant les mêmes caractéristiques que celles données ci-dessus.

Exemple 2

Préparation d'héparine N-acétylée-sursulfatée (IC 861746)

a. Préparation du sel de triéthylamine de l'héparine N-acétylée

L'héparine est N-désulfatée de façon classique (Inoue S. et Nagasawa K., Carbohyd, Res., 46 (1976), 87—95), puis acétylée par l'anhydride acétique en milieu aqueux basique. Le sel de TEA est ensuite préparé de façon identique à celle décrite dans l'exemple 1a.

b. Sulfatation

Le sel de TEA obtenu à partir d'un gramme d'héparine N-acétylée est dissous dans le DMF (25 ml) puis chauffé à 100°C en présence de $TMA/SO_3$ (0,84 g) pendant une nuit. Le produit sulfaté est récupéré après gel filtration sur Sephadex®G 25. Il est converti en sel de sodium après passage transitoire sous la forme acide. On obtient après lyophilisation 1,14 g de sel de sodium légèrement beige IC 861716. Sur les figures 4 et 5, on a représenté les spectres RMN pour l'héparine N-acétylée et pour le produit correspondant sursulfaté.

Exemple 3

Préparation d'héparine de bas poids moléculaire N-acétylée et sursulfatée

a. Préparation du sel de tétrabutylammonium (TAB) de l'héparine des bas PM N-acétylée.

On opère comme dans l'exemple 2a, mais en utilisant de l'hydroxyde de tétrabutylammonium comme base et une héparine de bas poids moléculaire.

b. Sulfatation

Le sel obtenu (1 g) est sulfaté par la complexe pyridine/$SO_3$ (1 g) dans la pyridine (10 ml à température ambiante pendant 24 heures). On obtient après gel filtration puis conversion en sel de sodium 0,5 g de sel de sodium d'héparine de bas poids moléculaire N-acétylée.

c. Caractérisation

Le procédé ci-dessus appliqué par exemple au produit CY 216 tel qu'obtenu selon l'exemple 1 du brevet FR—A—2440376 mentionné plus haut (dp moyen de l'ordre de 14) conduit à un produit sursulfaté (IC 841546) ayant un rapport $SO_3^-/COO^-$ de 2,85 celui du produit de départ étant de 2,12.

Exemple 4

Préparation d'héparine de bas poids moléculaire sursulfatée

a. Préparation du sel de tétrabutylammonim de l'héparine de bas PM CY 216

L'héparine de bas PM CY 216 est dissous dans l'eau (10 q dans 0,5 l) puis converti en acide par passage sur une colonne de résine Dowex® 50 WX4 sous forme $H^+$. Après neutralisation, l'héparine de tétrabutylammonium obtenue est lyophilisée. On obtient 19,34 g de sel de tétrabutylammonium.

b. Sulfatation

1 g du sel ci-dessus est dissous 50 ml de pyridine anhydre et on ajoute du complexe sulfatant (pyridine/$SO_3$). On procède à 3 essais avec des quantités différentes de complexe. A la fin de la réaction, 24 heures à température ambiante, on verse le mélange réactionnel dans une solution de soude (2 g) dans de l'éthanol à 95%. Le précipité formé est filtré, dissous dans l'eau, chromatographié sur Sephadex® G 25 et lyophilisé. On obtient les sels de sodium.

c. Caractérisation

En faisant varier la masse de complexe sulfatant, on fait varier le degré de sulfatation du produit formé. Ainsi, en utilisant respectivement des masses de complexe sulfatant de 0,16 g, 0,32 g et 0,48 g, on obtient des degrés de sulfatation de 2,21, 2,75 et 3,41.

Le spectre RMN du CY 216 sursulfaté est représenté sur la figure 3.

Le signal du Cl de l'anhydromannitol (63,6 ppm) a disparu ($CH_2$ OH→$CH_2O$ $SO_3^-$) de même les autres signaux du manitol (région 78,86 ppm) sont modifiés. Il en est de même des signaux des groupes anomères vers 100 ppm.

Exemple 5

Préparation d'oligosaccharides de degré de dépolymérisation, homogène à profil de sulfatation modifié

a. Préparation des oligosaccharides

Ils sont obtenus par fractionnement par gel filtration du mélange résultant de la dépolymérisation de l'héparine dans des conditions ménagées, par l'héparinase, ou par l'acide nitreux. Des procédés de dépolymérisation à l'aide d'acide nitreux sont décrits notamment dans le deuxième certificat d'addition 2478646 du 20.03.1980 et la demande FR 2503714 du 10.04.1981, dépolymérisation à l'aide d'héparinase est décrit dans la demande EP 0027089. Ces brevets et demandes de brevets, au nom de la demanderesse, sont évoqués plus haut. Le mélange de dépolymérisation utilisé (1 g) est déposé au sommet d'une colonne de gel de Sephadex® G 50 (2,5 × 300 cm) éluée par une solution de chlorure de sodium 0,2M. Les fractions correspondant à des oligosaccharides bien définis (di-, tétra-, hexa-, octa-, . . . jusqu'à eicosasaccharides) sont regroupées, concentrées, dessalées. Ces produits sont obtenus après lyophilisation.

b. Préparation des sels de tétrabutylammonium

Ils sont préparés comme décrit pour la préparation du sel en 4a. Les sels de tétra-, hexa-, octa-, dodéca- et hexadécasaccharides de produits terminés par des résidus d'acides uroniques insaturés situés à l'extrémité non réductrice ont été obtenus.

c. Sulfatation des oligosaccharides

Le sel de tétrabutylammonium de l'oligosaccharide à sulfater est dissous dans du DMF (250 mg/5 ml) puis du complexe sulfatant (triméthylamine/$SO_3$; 250 mg) est ajouté. On porte le mélange réactionnel à 50°C pendant 24 heures après dilution avec de l'eau (5 ml), l'oligosaccharide sulfaté est obtenu à la suite d'une chromatographie du mélange réactionnel sur Sephadex® G 25 suivie d'une lyophilisation.

| Oligosaccharide | dp | $SO_3^-/COO^-$ |
|---|---|---|
| Disaccharide | 2 | 3 |
| Disaccharide sursulfaté | 2 | 4,1 |
| Tétrasaccharide (IC841477) | 4 | 2,46 |
| Tétrasaccharide sursulfaté (IC851597) | 4 | 3,20 |
| Hexasaccharide (IC841476) | 6 | 2,22 |
| Hexasaccharide sursulfaté (IC851598) | 6 | 3,00 |
| Octasaccharide (IC841475) | 8 | 2,43 |
| Octasaccharide sursulfaté (IC851599) | 8 | 3,08 |
| Désacaccharide | 10 | 1,95 |
| Désaccharide sursulfaté | 10 | 2,76 |

Exemple 6

Préparation de dermatane-sulfate sursultaté (IC851610)

a. Préparation du sel de tétrabutylammonium

25 mg de dermatane-sulfate sont convertis en acide (résine échangeuse de cations) puis sont neutralisés par de l'hydroxyde de tétrabutylammonium. Après lyophilisation, on obtient 46 mg de sel.

b. Sursulfatation du dermatane-sulfate

Le sel obtenu ci-dessus (15 mg) est sursulfaté dans le DMF (1 ml) par addition de complexe triméthylamine/$SO_3$ (15 mg). Le produit est obtenu après dilution par l'eau, chromatographie sur gel de Sephadex® G 25 et enfin échange du cation organique pour du sodium au moyen d'une résine échangeuse d'ions.

Exemple 7

Préparation d'héparane-sulfate sursulfatée

a. Préparation du sel de triéthylamine

Le produit de départ (0,8 g) est dissous dans l'eau, converti en acide et neutralisé par la triéthylamine. On obtient ainsi après lyophilisation, 0.91 g de sel de triéthylamine.

b. Sursulfatation

Le sel ci-dessus (0,24 g) est dissous à chaud dans du DMF (8 ml). Après additions de complexe sulfatant ($TMA/SO_3$; 1,68 g), on maintient à 50°C pendant 24 heures. On obtient après gel filtration et conversion en sel de sodium, 238 mg d'héparine sursulfaté.

Exemple 8

Préparation d'héparine sursulfatée sur les positions 2 et 3 des motifs osidiques constitutifs

Cet exemple permet d'illustrer la souplesse du procédé. On prépare d'abord une héparine N,O-désulfatée selon la technique de NAGASAWA et al., Carbohyd. Res., 58 (1977) 47—55.

Cette héparine est ensuite N-resulfatée sélectivement par le complexe $pyridine/SO_3$ à pH basique. Elle est enfin convertie en sel de tétrabutylammonium par passage sous la forme acide et neutralisation par l'hydroxyde de tétrabutylammonium. Le sel ainsi obtenu est desséché à 50°C sous vide pendant 24 heures.

Le sel de tétrabutylammonium (0,92 g) est ensuite dissous dans de la pyridine anhydre (10 ml). On ajoute de l'anhydride acétique (0,1 ml) et laisse une nuit à température ambiante. Après addition de complexe sulfatant ($pyridine/SO_3$; 0,72 g), on chauffe le mélange réactionnel pendant 4 heures à 100°C. Aprés refroidissement, il est versé lentement dans de l'éthanol (100 ml) contenant de la soude (2 g). Après une nuit à 4°C, le précipité formé est essoré, dissous dans l'eau puis chromatographié sur Sephadex® G 25. Après lyophilisation, on obtient 0,67 g d'une poudre dont l'analyse révèle qu'environ 60% des fonctions alcool primaires sont libres.

Exemple 9

Préparation d'hexasaccharides N-acétyléssursulfatés

La fraction hexasaccharidique décrite dans l'example 5a est convertie en sel de pyridinium puis N-désulfatée par chauffage pendant 90 minutes à 50°C dans un mélange $DMSO/H_2O$ (95/5; v/v). Après addition de soude, le mélange réactionnel est chromatographié sur colonne de Sephadex® G 25. On récupère après lyophilisation, le produit sous forme d'amine libre. Il est N-acétylé de façon classique par l'anhydride acétique en milieu basique.

Le dérivé N-acétylé (0,5 g) est converti en sel de tétrabutylammonium. Il est ensuite dissous dans le DMF (10 ml) puis sulfaté à 100°C pendant 4 heures en présence de complexe $triméthylamine/SO_3$ (0,5 g). Après refroidissement, le mélange réactionnel est versé lentement dans une solution de soude 0,5 M dans l'éthanol (90 ml). Après centrifugation, le culot est récupéré. Il est dissous dans l'eau et lyophilisé. On obtient ainsi 0,61 g d'hexasaccharides N-acétylés, sursulfatés sous forme sel de sodium.

Exemple 10

Préparation de fragments de dermatanesulfate sursulfatés

Des fragments de dermatane-sulfate sont obtenus de façon classique (par oxydation periodique suivie d'hydrolyse ménagée acide: TOLLEFSEN, Nouvelle Revue Française d'Hématologie; 26 (1984) 233—237 ou bien par hydrazinolyse suivie de dégradation par l'acide nitreux, ou encore de la façon décrite ci-dessous).

Du dermatane-sulfate (produit Sigma), 50 mg, est dissous dans une solution 0,5 M d'acide chlorhydrique (2 ml). Après chauffage à 100°C pendant 5 minutes, le mélange est refroidi. On ajoute alors une solution de nitrite de sodium dans l'eau (2,5%; 1 ml). Après 1 minute, de la soude est ajoutée pour neutraliser l'acide suivi de borohydrure de sodium (10 mg) le mélange est dessalé sur une colonne de Sephadex® G 25.

Après lyophilisation, le produit obtenu est converti en sel de triéthylamine, puis il est sulfaté à 100°C dans le DMF (5 ml) en présence de complexe $triméthylamine/SO_3$ (50 mg). Après 4 heures, le mélange est refroidi puis déposé au sommet d'une colonne de Sepahdex® G 50 éluée par l'eau. Le sel de sodium est obtenu par passage intermédiaire sous la forme acide et neutralisation par de la soude. Cette neutralisation, suivie par conductimétrie, indique un rapport sulfate/carboxyle de 2,4.

Le temps d'hydrolyse, ou la température, ou encore la molarité de l'acide, peuvent être modifiés pour obtenir des fragments de taille différente. On peut également moduler le degré de sulfatation en changeant les conditions de cette réaction.

Cette réaction s'applique dans les mêmes conditions aux chondroïtines sulfates.

**Revendications**

1. Procédé de sulfatation de glycosaminoglycanes ou GAGs, caractérisé en ce qu'on transforme un GAG donné en un sel soluble dans un solvent organique qu'on le solubilise dans un solvant organique, et qu'on traite le sel en solution par un agent de sulfatation.

2. Procédé selon la revendication 1, caractérisé en ce que le GAG de départ est à base des motifs

alternés D-glucosamine-acide uronique, à savoir acide L-iduronique ou acide D-glucuronique.

3. Procédé selon la revendication 2, caractérisé en ce que le GAG de départ est choisi parmi l'héparine, l'héparane-sulfate, leurs fractions ou leurs fragments.

4. Procédé selon l'une des revendications 2 ou 3, caractérisé en ce que le GAG de départ est un GAG de bas poids moléculaire formé d'un mélange de chaînes, ou de chaînes homogènes au regard de leur degré de polymérisation, ayant un nombre de motifs sucre inférieur à celui de l'héparine ou de l'héparane-sulfate, pouvant varier de 2 à 30.

5. Procédé selon la revendication 4, caractérisé en ce que le GAG de départe est choisi parmi:

— les mucopolysaccharides tels qu'obtenus par extraction alcoolique à partir d'héparine, formés d'un mélange de chaînes de poids moléculaires d'environ 2000 à 8000 et possédant des rapports des titres Yin Wessler/USP d'au moins 2, tels que les produits possédant un rapport YW/USP de l'ordre de 3 à 5 et des poids moléculaires moyens de 3000 à 5500.

— les mucopolysaccharides tels qu'obtenus par dépolymérisation ménagée de l'héparine à l'aide d'acide nitreux, ces produits répondant d'une manière générale aux caractéristiques ci-dessus mais étant terminés par des groupes de structure 2,5-anhydro-manno, en particulier les mucopolysaccharides possédant une extrémité 2,5-anhydro-mannitol, ou encore une extrémité acide 2,5-anhydro-mannonique et formés de chaînes dont la majorité possède des poids moléculaires de l'ordre de 2000 à 3000, notamment d'environ 2000 à 2600, les rapports des titres Yin-Wessler/USP étant notamment d'au moins 10 avec des titres Yin-Wessler d'au moins environ 200 µ/mg,

— les mucopolysaccharides essentiellement formés de chaînes (1) d'un poids moléculaire moyen de 3000 à 6000 daltons, notamment de 4000 à 5000 environ (2) possédant un titre YW/USP inférieur à 10, notamment d'environ 6 à 3, (3) terminées par des motifs de structure 2,5-anhydromanno,

— les oligosaccharides formés de 8 motifs sucre au plus, de titre Yin-Wessler élevé pouvant atteindre 2000 µ/mg et de titre USP faible, pratiquement nul, dotés d'une forte affinité pour l'AT—III, ces oligosaccharides étant terminés par un groupe de structure 2,5-anhydro-manno ou comportant un motif de début de chaine correspondant à un motif uronique insaturé, tel que formé en soumettant l'héparine à une dépolymérisation ménagée à l'aide d'une héparinase, ces oligosaccharides présentant la structure ABCDEFGH de formule:

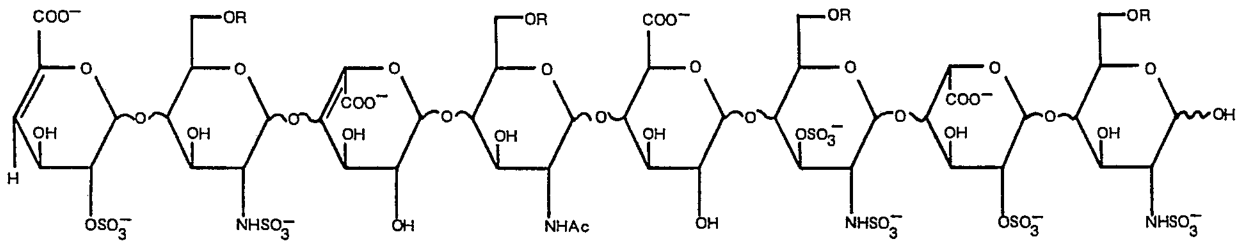

A B C D E F G H

— les compositions homogènes d'hexasaccharides, répondant à la formule ci-dessous

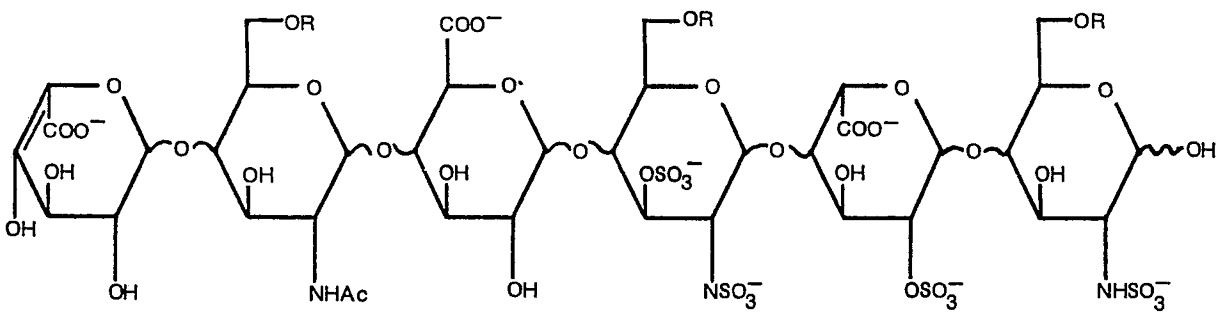

C' D E F G H

— les oligo ou polysaccharides de degré de polymérisation homogène, obtenus par filtration, sur un matériau tel que du Séphadex, par chromatographie par gradient de force ionique des GAGs ci-dessus.

— les GAGs tels qu'obtenus par action de périodate sur l'héparine, suivie de traitement en milieu basique, et le cas échéant d'une réduction par le borohydrure de sodium,

— les GAGs homogènes au regard de leur degré de polymérisation, tels qu'obtenus, par exemple, par filtration sur un gel d'un mélange de dépolymérisation d'héparine,

— les GAGs tels qu'obtenus par dépolymérisation de l'héparine avec de l'héparinase ou de l'acide nitreux, séparation des fractions possédant la séquence de fixation à l'AT—III et gel filtration de ces fractions afin d'obtenir d'une part des fragments de GAGs de degré de polymérisation varié, par exemple de fragments de dp supérieur à 12 d'une part, et d'autre part de fragments de GAGS de degré de polymérisation inférieure, notamment contenant 12, 10, 8, 6, 4 et 2 motifs sucre,

— les GAGS tels qu'évoqués ci-dessus mais pratiquement totalement dépourvus de la séquence capable de reconnaître spécifiquement l'AT—III et de titre YW faible à nul, ces GAGs ne fixant par sur l'AT—III dans les procédés comportant une étape de mise en contact d'héparine ou de GAGs avec de l'AT—III, suivie de gel filtration pour obtenir des fragments homogènes de dp varié.

— les GAGs, fractions et fragments de GAGs ci-dessus possédant des enchaînements tels que rencontrés dans l'héparine, dans lesquels une partie des groupes —$NHSO_3$ en position 2 des motifs glucosamine, voire la majorité et même la totalité, sont remplacés par des groupes —NH-acyle, en particulier —NH-acétyle, ou par des groupes $NH_2$.

—les GAGs dits totaux, correspondant aux compositions de GAGs obtenues par traitement d'organes animaux.

6. Procédé selon la revendication 1, caractérisé en ce que le GAG de départ est à base de motifs alternés D-galactosamine-acide uronique.

7. Procédé selon la revendication 6, caractérisé en ce que le GAG de départ est choisi parmi le dermatane-sulfate, les chondroïitines, les chondroïtines-sulfates ou encore l'acide hyaluronique, leurs fractions et leurs fragments.

8. Procédé selon l'une quelconque des revendications 1 à 7, caractérisé en ce qu'un ou plusieurs groupes —OH primaires ou secondaires des motifs saccharidiques de la chaîne glycosidique des GAGs mis en oeuvre sont bloqués par des groupements protecteurs.

9. Procédé selon la revendication 6 ou 7, caractérisé en ce que les GAGs de départ sont homogènes au regard de leur degré de polymérisation, ces GAGs étant obtenus, par exemple, par filtration sur un gel d'un mélange de dépolymérisation de ces GAGs.

10. Procédé selon l'une quelconque des revendications 1 à 9, caractérisé en ce que le sel de GAG mis en oeuvre est un sel d'amine.

11. Procédé selon la revendication 10, caractérisé en ce que le sel d'amine est choisi dans le groupe d'un sel d'une amine de formule —$N(R_1, R_2, R_3)$ avec $R_1$, $R_2$ et/ou $R_3$ représentant un atome d'hydrogène ou une chaîne aliphatique de 1 à 10 atomes de carbone, en particulier la triéthylamine ou la tributylamine ou encore d'un sel d'ammonium quaternaire.

12. Procédé selon l'une des revendications 1 à 11, caractérisé en ce que le sel de GAG mis en oeuvre est obtenu en soumettant le GAG sous forme acide obtenu par utilisation d'une résine échangeuse d'ions acides, à l'action d'une amine correspondant au sel qu'on souhaite former.

13. Procédé selon l'une quelconque des revendications 1 à 12, caractérisé en ce que la dissolution du sel de GAG est effectuée dans un solvant organique choisi parmi le diméthylformamide, le diméthylsulfoxyde ou la pyridine.

14. Procédé selon l'une quelconque des revendications 1 à 13, caractérisé en ce que l'agent de sulfatation est un complexe d'anhydride sulfurique $SO_3$ avec une base organique telle que la triméthylamine (TMA) ou la pyridine, ou de l'acide chlorosulfonique dans la pyridine.

15. Procédé selon la revendication 14, caractérisé en ce qu'on utilise environ 1 à 5 équivalents de complexe par équivalent de OH du GAG.

16. Procédé selon l'une quelconque des revendications 1 à 15, caractérisé en ce que la réaction de sulfatation est effectuée à une température de l'ordre de 0° à 100°C, notamment de l'ordre de 20°C ou supérieure pendant environ 10 à 14 heures.

17. Procédé selon l'une des revendications 1 et 14, caractérisé en ce qu'on élimine après l'étape de sulfatation les groupes protecteurs de radicaux hydroxyle —O-acyle et —O-benzoyle respectivement, par saponification ou par hydrogénation catalytique.

18. Procédé selon l'une quelconque des revendications 1 à 17, caractérisé en ce que le GAG sulfate obtenu est récupéré et soumis à une opération de gel filtration afin d'obtenir des GAGs homogènes au regard de leur degré de polymérisation.

19. GAGs à profil de sulfatation modifié, homogènes au regard de leur degré de polymérisation, ou en mélanges, charactérisés en ce qu'ils répondent à la formule I suivante:

$$R—(XY)_n—R'$$

dans laquelle:

R représente un acide uronique ou un acide uronique insaturé ou un groupe OH.

X représente une glucosamine ou une galactosamine dans laquelle le carbone en position 2 est substitué par un groupe $NHSO_3$, ou dans le cas d'une glucosamine et pour des chaînes de type héparinique par un groupe $NH_2$ ou NH-acyle.

Y représente un acide uronique, à savoir un acide D-glucuronique ou L-iduronique,

n est un nombre entier de 0 à 80,

R' représente une glucosamine ou une galactosamine dans laquelle le carbone en position 2 est substitué par un groupe $NHSO_3^-$, ou dans le cas de la glucosamine et pour des chaînes de type héparinique par un groupe $NH_2$, ou NH-acyle, ou encore représente un groupe —OH ou bien un motif glucosamine réarrangé en groupe de structure 2,5-anhydromanno ou représente un motif galactosamine réarrangé en groupe de structure 2,5-anhydrohexitol, étant entendu que lorsque, n = O, R et R' sont différents de OH, les groupes OH primaires ou secondaires des motifs X et Y, et, le cas échéant, de R et R', étant sulfatés de telle sorte que la position et/ou le nombre des groupes sulfate dans la chaîne de GAGs soient différents de ceux

rencontrés dans les chaînes naturelles ou dépolymérisées de GAGs correspondants, ou leurs sels pharmacologiquement acceptable, à l'exclusion des mélanges de chaînes à extrémités non modifiées ayant un nombre total de motifs glucosamine-acide uronique de 8 à 30.

20. Produits selon la revendication 19, caractérisés en ce qu'ils répondent à la formule II suivante:

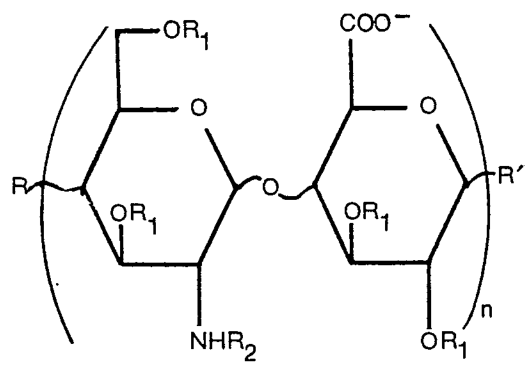

(II)

dans laquelle:

$R_1$ représente H ou $SO_3^-$

$R_2$ représente H, $SO_3^-$ ou un groupe acyle, notamment acétyle

R et R' ont les significations données ci-dessus.

21. GAGs selon la revendication 19, caractérisé en ce que X et le cas échéant R' représentent un motif galactosamine, les liaisons entre les motifs galactosamine-acide uronique étant du type 1→4 β,D et celles entre les motifs acide uronique-galactosamine éventuellement présents étant du type 1→3α, L ou 1→3 βD.

22. GAGs selon l'une quelconque des revendications 19 à 21, caractérisés en ce qu'ils renferment un nombre de motifs sucres inférieur ou égal à 12.

23. GAGs selon la revendication 22, caractérisés en ce que R représente un acide uronique insaturé.

24. GAGs selon la revendication 22, caractérisés en ce que R' représente un groupe de structure 2,5-anhydromanno.

25. GAGs selon la revendication 24, caractérisés en ce que R' représente un groupe de structure 2,5-anhydromannitol.

26. GAGs selon la revendication 22, caractérisés en ce qu'ils comportent un ensemble de GAGs homogènes au regard de degré de polymérisation dont les chaines ont quatre, six ou huit motifs sucre.

27. GAGs selon la revendication 26, caractérisés en ce que R et R' représentent soit simultanément OH, soit respectivement un acide uronique et une glucosamine.

28. GAGs selon la revendication 26, caractérisés en ce que R' représente un groupe de structure 2,5-anhydrohexitol.

29. GAGs selon la revendication 28, caractérisés en ce que R' représente un groupe de structure 2,5-anhydromannitol.

30. GAGs selon la revendication 22, caractérisés en ce que qu'ils comportent un ensemble homogène de GAGs dont les chaînes ont huit, dix ou douze motifs sucre.

31. GAGs selon la revendication 19, caractérisés en ce qu'ils renferment un mélange de chaînes répondant à la formule I dans laquelle n est un nombre entier de 3 à 15, et R et/ou R' représentent un motif sucre modifié.

32. GAGs selon la revendication 31, caractérisés en ce qu'ils sont préparés à partir de produits de départ constitués de mucopolysaccharides tels qu'obtenus par dépolymérisation ménagée à l'acide nitreux, formés d'un mélange de chaînes ayant un motif terminal 2,5-anhydromanno, en particulier 2,5-anhydromannitol, de poids moléculaires de l'ordre de 2000 à 8000, possédant un rapport des titres Yin-Wessler/USP d'au moins 3, un titre Yin-Wessler d'au moins 200 µ/mg et des poids moléculaires moyens de 2000 à 3000.

33. GAGs selon la revendication 31, caractérisés en ce qu'ils sont préparés à partir de produits de départ constitués de mucopalysaccharides tels qu'obtenus par dépolymérisation ménagée à l'acide nitreux, formés d'un mélange de chaînes ayant un motif terminal 2,5 anhydromanno, en particulier 2,5 anhydromannitol, possédant un rapport des titres Yin-Wessler/USP d'environ 3 à 6 et des poids moléculaires moyens de 4000 à 5000.

34. GAGs selon la revendication 31, caractérisés en ce que le carbone en position 2 de la glucosamine et substitué par un groupe $NH_2$ ou NH-acyle, notamment NH-acétyle.

35. GAGs selon la revendication 19, caractérisés en ce qu'ils renferment un nombre de motifs sucre supérieur à 30.

36. GAGs selon la revendication 35, caractérisés en ce qu'ils sont des produits naturels sursulfatés, à l'exception de l'héparine.

37. GAGs selon la revendication 36, caractérisés en ce que le carbone en position 2 de la glucosamine est substitué par un groupe $NH_2$ ou NH-acyle, notamment NH-acétyle.

38. Principe actif de médicament, caractérisés en ce qu'il est constitué par au moins un GAG à profil de sulfatation modifié selon la revendication 19.

39. Composition pharmaceutique, caractérisée en ce qu'elle renferme une quantité efficace d'au moins un GAG selon l'une quelconque des revendications 19 à 37 en association avec un véhicule pharmaceutique.

40. Composition selon la revendication 39, caractérisée en ce qu'elle se présente sous la forme d'une solution injectable stérile, contenant de 1 à 200 mg de GAGs, de préférence de 20 à 150 mg/ml, lorsque ces solutions sont destinées à l'injection sous-cutanée, ou de 30 à 100 mg/ml, not amment de 40 à 50 mg/ml de GAGs lorsqu'elles sont destinées à l'injection par voie intraveineuse ou par perfusion.

41. Composition selon la revendication 39, caractérisée en ce qu'elle se présente sous une forme administrable par voie orale telles que gélules gastrorésistantes, comprimés ou tablettes, pilules, ou liposomes.

42. Composition selon la revendication 39, caractérisée en ce qu'elle se présente sous forme de suppositoire.

43. Réactif biologique, caractérisé en ce qu'il comprend un GAG à profil de sulfatation modifié selon l'une quelconque des revendications 19 à 37.

**Patentansprüche**

1. Verfahren zur Sulfatierung von Glykosaminoglykanen oder GAGs, dadurch gekennzeichnet, daß man ein gegebenes GAG in ein in einem organischen Lösungsmittel lösliches Salz überführt, daß man es in einem organischen Lösungsmittel solubilisiert und daß man des Salz in Lösung mit einem Sulfatierungsmittel behandelt.

2. Verfahren nach Anspruch 1, dadurch gekennzeichnet, daß das Ausgangs-GAG auf alternierenden Einheiten von D-Glucosamin-Uronsäure, nämlich L-Iduronsäure oder D-Glucuronsäure, basiert.

3. Verfahren nach Anspruch 2, dadurch gekennzeichnet, daß das Ausgangs-GAG aus Heparin, Heparinsulfat, ihren Fraktionen oder ihren Fragmenten ausgewählt wird.

4. Verfahren nach einem der Ansprüche 2 oder 3, dadurch gekennzeichnet, daß das Ausgangs-GAG eine GAG mit niedrigem Molekulargewicht ist, das aus einer Mischung von Ketten oder hinsichtlich ihres Polymerisationsgrades homogenen Ketten gebildet wird, die eine Zahl von Zuckereinheiten besitzen, die kleiner ist als diejenige des Heparins oder des Heparinsulfats und die zwischen 2 und 30 liegen kann.

5. Verfahren nach Anspruch 4, dadurch gekennzeichnet, daß das Ausgangs-GAG ausgewählt wird aus:

— Mucopolysacchariden, wie sie durch alkoholische Extraktion ausgehend von Heparin erhalten werden, wobei sie eine Mischung von Ketten mit Molekulargewichten von etwa 2000 bis 8000 bilden und Yin Wessler/USP Titerverhältnisse von mindestens 2 besitzen, wie die Produkte, die ein YW/USP-Verhältnis von etwa 3 bis 5 und mittlere Molekulargewichte von 3000 bis 5500 besitzen;

— Mucopolysacchariden, wie sie durch schonende Depolymerisation von Heparin mit Hilfe von salpetriger Säure erhalten werden, wobei diese Produkte im allgemeinen den obigen Charakteristika entsprechen, aber endständige Gruppen mit der 2,5-Anhydro-manno-Struktur aufweisen, insbesondere Mucopolysacchariden, die eine endständige 2,5-Anhydro-mannit-Gruppe oder auch eine endständige 2,5-Anhydro-mannonsäure-Gruppe besitzen und aus Ketten aufgebaut sind, deren Mehrzahl Molekulargewichte von etwa 2000 bis 3000, insbesondere etwa 2000 bis 2600, besitzen, wobei die Yin-Wessler/USP-Titerverhältnisse insbesondere mindestens 10 betragen, bei Yin-Wessler-Titern von mindestens 200 µ/mg;

— Mucopolysacchariden, die im wesentlichen aus Ketten gebildet werden mit (1) einem mittleren Molekulargewicht von 3000 bis 6000 Dalton, insbesondere etwa 4000 bis 5000, (2) einem YW/USP-Titer unterhalb von 10, insbesondere etwa 6 bis 3, und die (3) durch Einheiten mit der 2,5-Anhydro-manno Struktur abgeschlossen sind;

— Oligosacchariden, die aus höchstens 8 Zuckereinheiten gebildet werden, mit erhöhtem Yin-Wessler-Titer, der 2000 µ/mg erreichen kann und mit einem schwachen USP-Titer, praktisch von Null, versehen mit einer starken Affinität zu AT—III, wobei diese Oligosaccharide durch eine Gruppe mit der 2,5-Anhydromanno-Struktur abgeschlossen sind oder eine Anfangsketteneinheit entsprechend einer ungesättigten Uronsäure-Einheit aufweisen, wie sie gebildet werden, wenn man Heparin einer milden Depolymerisation mit Hilfe von Heparinase unterwirft, wobei diese Oligosaccharide die Struktur ABCDEFGH der Formel besitzen:

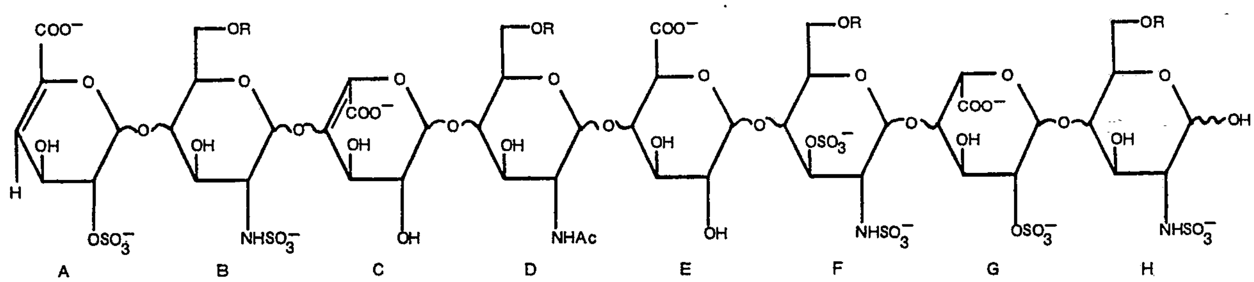

— homogenen Zusammensetzungen von Hexasacchariden entsprechend der nachstehenden Formel

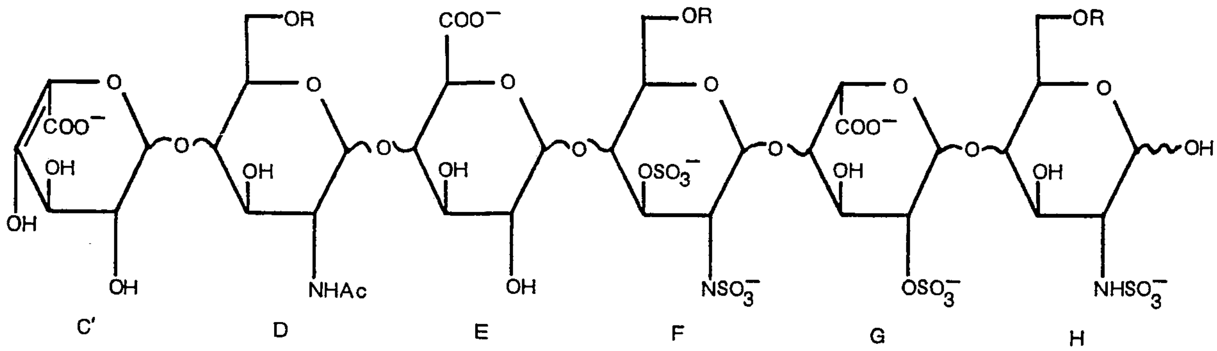

—Oligo- oder Polysacchariden mit homogenem Polymerisations- grad, erhalten aus den obigen GAGs durch Filtration über ein Material, wie Sephadex, oder durch Ionen-Hochleistungs-Gradient-Chromatographie;

—GAGs, wie sie erhalten werden durch Perjodateinwirkung auf Heparin, gefolgt von einer Behandlung im basischen Milieu und gegebenenfalls einer Reduktion durch Natriumborhydrid;

— GAGs, die hinsichtlich des Polymerisationsgrades homogen sind, wie sie zum Beispiel erhalten werden durch Filtration einer Mischung von depolymerisiertem Heparin durch ein Gel;

—GAGs, wie sie erhalten werden durch Depolymerisation von Heparin mit Heparinase oder salpetriger Säure, Trennung der Fraktionen, die die Sequenz der Bindung an AT—III besitzen, und Gelfiltration dieser Fraktionen, um Fragments der GAGs mit unterschiedlichem Polymerisationsgrad zu erhalten, zum Beispiel einerseits Fragmente mit einem Polymerisationsgrad von größer als 12, und anderseits Fragmente der GAGs mit niedrigerem Polymerisationsgrad, inbesondere 12, 10, 8, 6, 4 und 2-Zuckereinheiten enthaltend;

— GAGs, wie oben erwähnt, aber praktisch vollständig ohne Sequenz, die zur spezifischen Erkennung von AT—III fähig ist, und mit einem YW-Titer, der schwach bis Null ist, wobei diese GAGs sich nicht an AT—III binden in den Verfahren, die eine Stufe enthalten, bei der Heparin oder GAGs mit AT—III in Kontakt gebracht werden, gefolgt von Gelfiltration, um homogene Fraktionen mit unterschiedlichem Polymerisationsgrad zu erhalten;

— obigen GAGs und ihren Fraktionen und Fragmenten die Verkettungen aufweisen, wie man sie in Heparin findet, in denen ein Teil der $NHSO_3$-Gruppen in 2-Stellung der Glucosamin-Einheiten, ja sogar die Mehrzahl oder sogar die Gesamtheit, durch NH-Acyl-Gruppen insbesondere NH-Acetyl, oder $NH_2$-Gruppen ersetzt sind;

— den angegebenen GAGs, die Zusammensetzungen von GAGs entsprechen, die durch Behandlung tierischer Organe erhalten werden.

6. Verfahren nach Anspruch 1, dadurch gekennzeichnet, daß das Ausgangs-GAG auf alternierenden Einheiten von D-Galactosamin-Uronsäure basiert.

7. Verfahren nach Anspruch 6, dadurch gekennzeichnet, daß das Ausgangs-GAG aus Dermatansulfat, Chondroitinen, Chondroitin-sulfaten, oder auch Hyaluronsäure, ihren Fraktionen und ihren Fragmenten ausgewählt wird.

8. Verfahren nach einem der Ansprüche 1 bis 7, dadurch gekennzeichnet, daß eine oder mehrere primäre oder sekundäre OH-Gruppen der Saccharid-Einheiten der glykosidischen Kette der eingesetzten GAGs durch Schutzgruppen blockiert sind.

9. Verfahren nach Anspruch 6 oder 7, dadurch gekennzeichnet, daß die Ausgangs-GAGs hinsichtlich ihres Polymerisationsgrades homogen sind, wobei diese GAGs zum Beispiel erhalten werden durch Filtration einer Depolymerisationsmischung dieser GAGs durch ein Gel.

10. Verfahren nach einem der Ansprüche 1 bis 9, dadurch gekennzeichnet, daß das eingesetzte Salz des GAGs ein Aminsalz ist.

11. Verfahren nach Anspruch 10, dadurch gekennzeichnet, daß das Aminsalz ausgewählt wird aus der Gruppe eines Aminsalzes oder Formel —$N(R_1, R_2, R_3)$, wobei $R_1$, $R_2$ und/oder $R_3$ ein Wasserstoffatom oder eine aliphatische Kette mit 1 bis 10 Kohlenstoffatomen bedeuten, insbesondere Triäthylamin oder Tributylamin, oder auch ein quaternäres Ammoniumsalz.

12. Verfahren nach einem der Ansprüche 1 bis 11, dadurch gekennzeichnet, daß das Salz des eingesetzten GAGs erhalten wird, indem man das GAG in Form der durch Benützung eines sauren Ionenaustauscherharzes erhaltenen Säure der Wirkung eines Amins unterwirft, das dem Salz entspricht, das man zu erhalten wünscht.

13. Verfahren nach einem der Ansprüche 1 bis 12, dadurch gekennzeichnet, daß die Auflösung des Salzes des GAGs in einem organischen Lösungsmittel, ausgewählt aus Dimethylformamid, Dimethylsulfoxid oder Pyridin, durchgeführt wird.

14. Verfahren nach einem der Ansprüche 1 bis 13, dadurch gekennzeichnet, daß das Sulfatierungsmittel ein Komplex von Schwefelsäureanhydrid $SO_3$ mit einer organischen Base, wie Trimethylamin (TMA) oder Pyridin, oder von Chlorsulfonsäure in Pyridin ist.

15. Verfahren nach Anspruch 14, dadurch gekennzeichnet, daß man etwa 1 bis 5 Äquivalente des Komplexes pro OH-Äquivalent des GAGs verwendet.

16. Verfahren nach einem der Ansprüche 1 bis 15, dadurch gekennzeichnet, daß die Sulfatierungsreaktion bei einer Temperatur von etwa 0° bis 100°C, insbesondere etwa 20°C oder höher, während etwa 10 bis 14 Stunden durchgeführt wird.

17. Verfahren nach einem der Ansprüche 1 bis 14, dadurch gekennzeichnet, daß man nach der Sulfatierungsstufe die Schutzgruppen der Hydroxyl-, O-Acyl- bzw. O-Benzoylreste durch Verseifen oder durch katalytische Hydrierung entfernt.

18. Verfahren nach einem der Ansprüche 1 bis 17, dadurch gekennzeichnet, daß das erhaltene GAG-Sulfat gewonnen und einer Gelfiltrationsoperation unterworfen wird, um GAGs zu erhalten, die in bezug auf ihren Polymerisationsgrad homogen sind.

19. GAGs mit einem modifizierten Sulfatierungsprofil, hinsichtlich ihres Polymerisationsgrades homogen oder in Form von Mischungen, dadurch gekennzeichnet, daß sie der folgenden Formel I entsprechen:

$$R—(XY)_n—R' \qquad (I)$$

worin:

R ein Uronsäure oder eine ungesättigte Uronsäure oder eine OH-Gruppe bedeutet;

X ein Glucosamin oder ein Galactosamin bedeutet, in dem das Kohlenstoffatom in 2-Stellung durch eine $NHSO_3$-Gruppe oder im Falle eines Glucosamins und hinsichtlich der Ketten vom Heparintyp durch eine $NH_2$— oder NH-Acyl-Gruppe substituiert ist;

Y eine Uronsäure, nämlich eine D-Glucuron- oder L-Iduronsäure bedeutet;

n für eine ganze Zahl von 0 bis 80 steht;

R' ein Glucosamin oder ein Galactosamin beduetet, in dem der Kohlenstoff in 2-Stellung durch eine $NHSO_3$-Gruppe substituiert ist oder im Falle eines Glucosamins und hinsichtlich der Ketten vom Heparintyp durch eine $NH_2$- oder NH-Acyl-Gruppe substituiert ist, oder eine OH-Gruppe oder eine Glucosamin-Einheit bedeutet, die in eine Gruppe mit der 2,5-Anhydro-manno-Struktur umgewandelt ist, oder eine Glucosamin-Einheit bedeutet, die in eine Gruppe mit der 2,5-Anhydro-hexit-Struktur umgewandelt ist; wobei es klar ist, daß dann, wenn n 0 ist, R und R' von OH verschieden sind; wobei die primären oder sekundären OH-Gruppen der Einheiten X und Y und gegebenenfalls von R und R' derart sulfatiert sind, daß die Stellung und/oder die Zahl der Sulfatgruppen in der Kette der GAGs verschieden sind von denjenigen, die man in natürlichen oder depolymerisierten Keten der entsprechenden GAGs vorfindet; oder ihre pharmakologisch verträglichen Salze, mit Ausnahme der Mischungen von Ketten, die an den Enden nicht modifiziert sind und eine Gesamtzahl von Glucosamin-Uronsäure-Einheiten von 8 bis 30 aufweisen.

20. Produkte nach Anspruch 19, dadurch gekennzeichnet, daß sie der folgenden Formel II entsprechen:

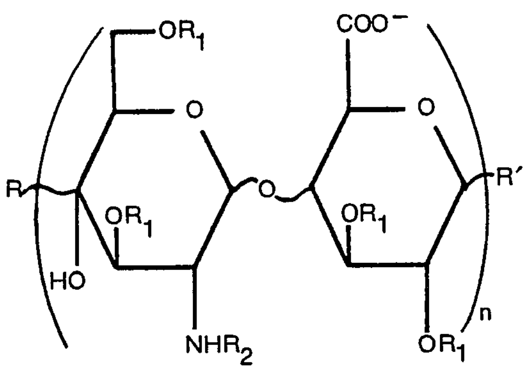

(II)

worin

$R_1$ H oder $SO_3$— bedeutet,

$R_2$ H, $SO_3$— oder eine Acylgruppe, insbesondere Acetyl bedeutet und

R und R' die oben angegebenen Bedeutungen besitzen.

21. GAGs nach Anspruch 19, dadurch gekennzeichnet, daß X und gegebenenfalls R' eine Galactosamin-Einheit darstellen, wobei die Bindungen zwischen den Galactosamin-Uronsäure-Einheiten vom Typ 1→4 β, D und diejenigen zwischen den gegebenenfalls vorhandenen Uronsäure-Galactosamin-Einheiten vom Typ 1→3 α,L oder 1→3 β,D sind.

22. GAGs nach einem der Ansprüche 19 bis 21, dadurch gekennzeichnet, daß sie eine Zahl von Zuckereinhieten unterhalb oder gleich 12 enthalten.

23. GAGs nach Anspruch 22, dadurch gekennzeichnet, daß R eine ungesättigte Uronsäure bedeutet.

24. GAGs nach Anspruch 22, dadurch gekennzeichnet, daß R' eine Gruppe mit der 2,5-Anhydro-manno-Struktur bedeutet.

25. GAGs nach Anspruch 24, dadurch gekennzeichnet, daß R' eine Gruppe mit der 2,5-Anhydro-mannit-Struktur bedeutet.

26. GAGs nach Anspruch 22, dadurch gekennzeichnet, daß sie eine Gesamtheit von hinsichtlich des Polymerisationsgrades homogenen GAGs bilden, deren Ketten vier, sechs oder acht Zuckereinheiten aufweisen.

27. GAGs nach Anspruch 26, dadurch gekennzeichnet, daß R und R' entweder gleichzeitig OH oder Uronsäure bzw. Glucosamin darstellen.

28. GAGs nach Anspruch 26, dadurch gekennzeichnet, daß R' eine Gruppe mit der 2,5-Anhydro-hexit-Struktur darstellt.

29. GAGs nach Anspruch 28, dadurch gekennzeichnet, daß R' eine Gruppe mit der 2,5-Anhydro-mannit-Struktur darstellt.

30. GAGs nach Anspruch 22, dadurch gekennzeichnet, daß sie eine homogene Gesamtheit von GAGs bilden, deren Ketten acht, zehn oder zwölf Zuckereinheiten aufweisen.

31. GAGs nach Anspruch 19, dadurch gekennzeichnet, daß sie eine Mischung von Ketten enthalten, die der Formel I entsprechen, worin n eine ganze Zahl von 3 bis 15 und R und/oder R' eine modifizierte Zuckereinheit bedeuten.

32. GAGs nach Anspruch 31, dadurch gekennzeichnet, daß sie aus Ausgangsmaterialien hergestellt wurden, die aus Mucopolysacchariden zusammengesetzt sind, wie sie durch milde Depolymerisation mit salpetriger Säure erhalten werden, und sich aus einer Mischung von Ketten zusammensetzen, die eine endständige 2,5-Anhydro-manno-, insbesondere 2,5-Anhydromannit-Einheit, ein Molekulargewicht von etwa 2000 bis 8000, ein Yin/Wessler/USP Titerverhältnis von mindestens 3, einen Yin/Wessler Titer von mindestens 200 µ/mg und mittlere Molekulargewichte von 2000 bis 3000 aufweisen.

33. GAGs nach Anspruch 31, dadurch gekennzeichnet, daß sie aus Ausgangsmaterialien hergestellt wurden, die aus Mucopolysacchariden zusammengesetzt sind, wie sie durch milde Depolymerisation mit salpetriger Säure erhalten werden, und sich aus einer Mischung von Ketten zusammensetzen, die eine endständige 2,5-Anhydro-manno-, insbesondere 2,5-Anhydromannit-Einheit, einem Yin/Wessler/USP Titerverhältnis von etwa 3 bis 6 und mittlere Molekulargewichte von 4000 bis 5000 aufweisen.

34. GAGs nach Anspruch 31, dadurch gekennzeichnet, daß der Kohlenstoff in 2-Stellung des Glucosamins durch eine $NH_2$- oder NH-Acyl-, insbesondere NH-Acetyl-Gruppe substituiert ist.

35. GAGs nach Anspruch 19, dadurch gekennzeichnet, daß sie eine Anzahl von Zuckereinheiten oberhalb von 30 besitzen.

36. GAGs nach Anspruch 35, dadurch gekennzeichnet, daß sie natürliche übersulfatierte Produkte sind, ausgenommen Heparin.

37. GAGs nach Anspruch 36, dadurch gekennzeichnet, daß der Kohlenstoff in 2-Stellung des Glucosamins durch eine $NH_2$- oder NH-Acyl-, insbesondere NH-Acetyl-Gruppe substituiert ist.

38. Arzneistoff, dadurch gekennzeichnet, daß er aus mindestens einem GAG mit modifiziertem Sulfatierungsprofil nach Anspruch 19 zusammengesetzt ist.

39. Arzneimittel, dadurch gekennzeichnet, daß zeichnet, daß es eine wirksame Menge von mindestens einem GAG nach einem der Ansprüche 19 bis 37 zusammen mit einem pharmazeutischen Träger enthält.

40. Arzneimittel nach Anspruch 39, dadurch gekennzeichnet, daß es in Form einer injizierbaren, sterilen Lösung vorliegt, die 1 bis 200 mg GAGs, vorzugsweise 20 bis 150 mg/ml, falls diese Lösungen für die subkutane Injektion vorgesehen sind, oder 30 bis 100 mg/ml, insbesondere 40 bis 50 mg/ml GAGs, falls sie für die intravenöse Injektion oder Perfusion vorgesehen sind, enthält.

41. Arzneimittel nach Anspruch 39, dadurch gekennzeichnet, daß es in einer Form für die orale Verabreichung vorliegt, wie magensaftresistente Kapseln, Tabletten, Pillen oder Liposomen.

42. Arzneimittel nach Anspruch 39, dadurch gekennzeichnet, daß es in Suppositorienform vorliegt.

43. Biologisches Reagens, dadurch gekennzeichnet, daß es ein GAG mit einem modifizierten Sulfatierungs-Profil nach einem der Ansprüche 19 bis 37 enthält.

**Claims**

1. Procedure for the sulfation of glycosaminoglycans or GAGs, characterized in that a given glycosaminoglycan is converted into a salt soluble in an organic solvent, that is is solubilized in an organic solvent and that the salt is treated with a sulfating agent.

2. Procedure according to claim 1, characterized in that the starting GAG is composed of alternating residues of D-glucosamine-uronic acid, namely, L-iduronic acid or D-glucuronic acid.

3. Procedure according to claim 2, characterized in that the starting GAG is chosen from among heparin, heparan sulfate, fractions thereof or fragments thereof.

4. Procedure according to one of the claims 2 or 3, characterized in that the starting GAG is a GAG of low molecular weight composed of a mixture of chains, or of chains homogeneous with respect to their degree of polymerisation, having a number of sugar residues less than that of heparin or heparan sulfate, which may vary from 2 to 30.

5. Procedure according to claim 4, characterized in that the starting GAG is chosen from among

— mucopolysaccharides such as those obtained from heparin by alcoholic extraction, composed of a mixture of chains of molecular weights of 2,000 to 8,000, and possessing ratios of Yin Wessler/USP titers of at least 2, such as substances possessing a YW/USP ratio of the order of 3 to 5 and average molecular weights of 3,000 to 5,500,

— mucopolysaccharides such as those obtained by limited depolymerisation of heparin by nitrous acid, these substances having the same general characteristics as those mentioned above but being terminated by residues with the 2,5-anhydromanno structure, including mucopolysaccharides possessing

a terminal 2,5-anhydromannitol residue, or a terminal 2,5-anhydromannonic acid residue, composed of chains, the majority of which possess molecular weights of the order of 2,000 to 3,000, particularly of 2000 to 2600, the ratios of Yin-Wessler/USP titers being at least 10, with Yin-Wessler titers of at least 200 µ/mg.

— mucopolysaccharides composed essentially of chains (1) of average molecular weight of from 3000 to 6000 daltons, possessing a YW/USP ratio of less than 10, and terminated by residues of the 2,5-anhydromanno structure,

— oligosaccharides composed of maximally 8 sugar residues, with a high Yin-Wessler titer which may rise to 2,000 µ/mg and a low USP titer, almost zero, endowed with a high affinity for AT—III, these oligosaccharides being terminated by a residue with the 2,5-anhydromanno structure or bearing a residue at the beginning of the chain corresponding to an unsaturated uronic acid residue, such as the formed by subjecting heparin to a limited depolymerisation with heparinase, said oligosaccharides having the structure ABCDEFGH of formula:

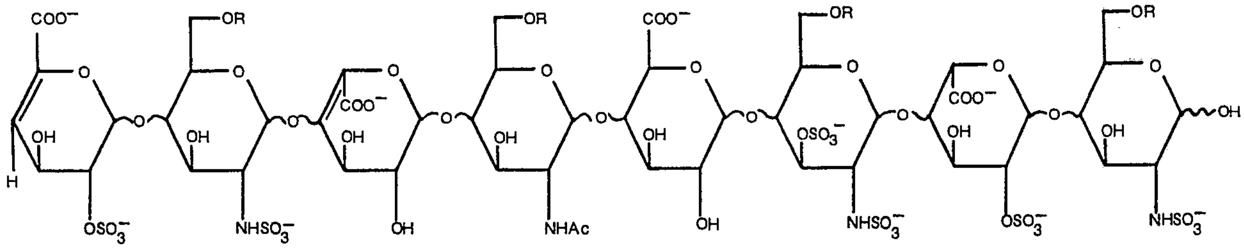

A B C D E F G H

— homogeneous hexasaccharide compositions having formula below:

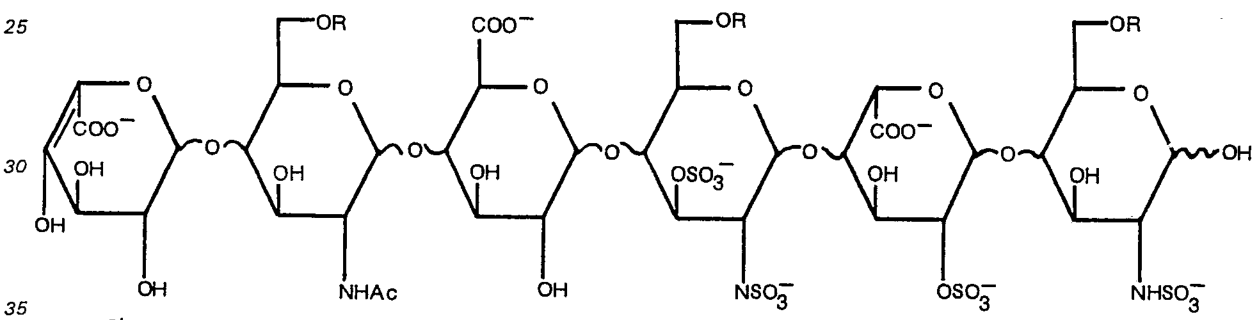

C' D E F G H

— oligo- or polysaccharides, homogeneous with respect to degree of polymerisation, obtained by filtration through a material such as Sephadex by chromatography employing an ionic strength gradient of the above GAGs,

— GAGs such as those obtained by the action of periodate on heparin, followed by treatment with base, and, if necessary, reduction by sodium borohydride,

— GAGs homogeneous with respect to degree of polymerization, such as obtained, for example, by gel filtration of a depolymerization mixture of heparin,

— GAGs such as those obtained by depolymerisation of heparin by heparinase or nitrous acid, the separation of the fractions possessing the sequence which binds at AT—III and the gel filtration of these fractions in order to obtain fragments of GAGs exhibiting varying degrees of polymerisation, for example, fragments, with a dp higher than 12, on the one hand, and on the other, GAG fragments having a lower degree of polymerisation, including those containing 12, 10, 8, 6, 4 and 2 sugar residues.

— GAGs such as those mentioned above but almost totally lacking the sequence able to specifically recognise AT—III and with YW titers varying from low to zero, including oligosaccharides which do not bind to AT—III in the procedures which include a step in which heparin or GAGs are mixed with AT—III followed by gel filtration to separate the oligosaccharides of various dps into homogeneous fragments,

— the GAG fractions of fragments thereof of GAGs mentioned above possessing chain sequences such as those encountered in heparin, in which some, even the majority and possibly all of the —$NHSO_3$ groups in position 2 of the glucosamine residues are replaced by —NH-acyl groups, including —NH-acetyl groups, or by —$NH_2$ groups,

— so-called total GAGs corresponding to mixtures of GAGs obtained by treatment of animal organs.

6. Procedure according to claim 1, characterized in that the starting GAG is composed of alternating residues of D-galactosamine and a uronic acid.

7. Procedure according to claim 6, characterized in that the starting GAG is chosen from among dermatan sulfate, the chondroitins, the chondroitin sulfates or even hyaluronic acid, fractions thereof or fragments thereof.

8. Procedure according to any one of the claims 1 to 7, characterized in that one or several primary or secondary —OH groups of the sugar residues of the glycosidic chain of the GAGs used are blocked by protecting groups.

9. Procedure according to claim 6 or 7, characterized in that the starting GAGs are homogeneous with respect to their degree of polymerisation, these GAGs being obtained, for example, by gel filtration of a depolymerised mixture of these GAGs.

10. Procedure according to any one of the claims 1 to 9, that the salt of the GAG used is an amine salt.

11. Procedure according to claim 10, characterized in that the amine salt is chosen from a group of amine salt of the formula —$N(R_1, R_2, R_3)$ in which $R_1$, $R_2$ and/or $R_3$ represent a hydrogen atom or an aliphatic chain of from 1 to 10 carbon atoms, including triethylamine or tributylamine or a quaternary ammonium salt.

12. Procedure according to one of the claims 1 to 11, characterized in that the GAG salt used is obtained by treating the GAG in its acid form, prepared by chromatography by using acid ions exchange resin, with the amine corresponding to the desired salt.

13. Procedure according to any one of the claims 1 to 12, characterized in that the GAG salt is dissolved in an organic solvent chosen from among dimethylformamide, dimethylsulfoxide or pyridine.

14. Procedure according to any one of the claims 1 to 13, characterized in that the sulfating agent is a complex of sulfur trioxide, $SO_3$, with an organic base such as trimethylamine (TMA) or pyridine, or chlorosulfonic acid in pyridine.

15. Procedure according to claim 14, characterized in that about 1 to 5 equivalents of complex are used per —OH group of the GAG.

16. Procedure according to any one of the claims 1 to 15, characterized in that the sulfation reaction is carried out at a temperature of the order of 0° to 100°C, preferably at about 20°C or above for 10 to 14 hours.

17. Procedure according to the claims 1 to 14, characterized in that, after the sulfation step, the O-acyl and O-benzoyl hydroxyl protecting groups are removed by saponification, or catalytic hydrogenation.

18. Procedure according to any one of the claims 1 to 17, characterized in that the sulfated GAG obtained is recovered and subjected to a gel filtration step in order to obtain GAGs homogeneous with respect to their degree of polymerisation.

19. GAGs with an altered sulfation pattern, homogeneous with respect to their degree of polymerisation, or as mixtures, characterized in that they are represented by the following formula I:

$$R—(XY)_n—R' \qquad (I)$$

in which:

R represents a uronic acid or an unsaturated uronic acid or an OH group.

X represents a glucosamine or a galactosamine moiety in which the carbon atom at position 2 is substituted by a $NHSO_3^-$ group, or, in the case of glucosamine and chains of the heparin type, by an $NH_2$ or NH-acyl group.

Y represents a uronic acid, namely D-glucuronic acid or L-iduronic acid.

n is an integer between 0 and 80.

R' represents a glucosamine or galactosamine moiety in which the carbon atom at position 2 is substituted by a $NHSO_3^-$ group, or, in the case of glucosamine and chains of the heparin type, by an $NH_2$ or NH-acyl group, or represents an —OH group or a glucosamine residue rearranged to a grouping with a 2,5-anhydromanno structure, or a galactosamine residue rearranged to a grouping with a 2,5-anhydrohexitol structure, with the proviso that when n = O, R and R' are different from OH, the primary and secondary OH groups of the residues X and Y and, if the case arises, of R and R', being sulfated such as the position and/or number of sulfate groups in the chain of the GAGs being different from those encountered in the naturally occurring chains or the depolymerised chains of corresponding GAGs, or their pharmacologically acceptable salts, excluding mixtures of chains with non-modified terminal having a total number of glucosamine and uronic acid residues of from 8 to 30.

20. Substances according to claim 19, characterized in that they correspond to formula II as follows:

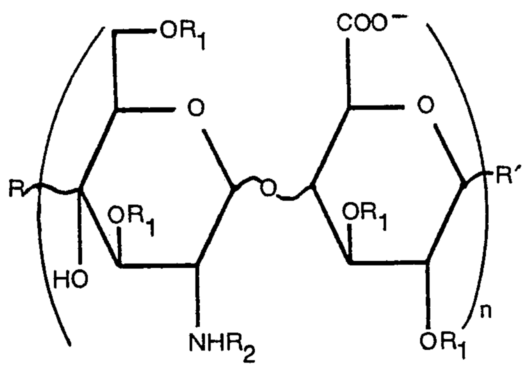

(II)

in which:

$R_1$ represents H or $SO_3^-$

$R_2$ represents H, $SO_3^-$ or an acyl group, in particular acetyl R and R' have the meanings given above.

21. GAGs according to claim 19, characterized in that X and possibly R′ represent a galactosamine residue, the linkages between the galactosamine and uronic acid residues being of the 1→4 β,D type and those between the uronic acid and the galactosamine residues possibly present being of the type 1→3α,L or 1→3β,D.

22. GAGs according to any one of the claims 19 or 21, characterized in that they contain a number of sugar residues less than or equal to 12.

23. GAGs according to the claim 22, characterized in that R represents an unsaturated uronic acid residue.

24. GAGs according to claim 22, characterized in that R′ represents a group with the 2,5-anhydromanno structure.

25. GAGs according to claim 24, characterized in that R′ represents a residue with the 2,5-anhydromannitol structure.

26. GAGs according to claim 22, characterized in that they comprise GAGs homogeneous with respect to degree of polymerization, the chains containing four, six or eight sugar residues.

27. GAGs according to claim 26, characterized in that R and R′ either both represent an OH or are respectively a uronic acid and a glucosamine residue.

28. GAGs according to claim 26, characterized in that R′ represents a residue with the 2,5-anhydrohexitol structure.

29. GAGs according to claim 28, characterized in that R′ represents a residue with the 2,5-anhydromannitol structure.

30. GAGs according to claim 22, characterized in that they comprise homogeneous GAGs the chains of which have eight, ten or twelve sugar residues.

31. GAGs according to claim 19, characterized in that they contain a mixture of chains represented by the formula I in which n is an integer between 3 and 15, and R and/or R′ represents a modified sugar residue.

32. GAGs according to claim 31, characterized in that they are prepared from starting materials consisting of mucopolysaccharides such as those obtained by limited depolymerisation with nitrous acid, composed of a mixture of chains with a terminal 2,5-anhydromanno residue, including a 2,5-anhydromannitol residue, of molecular weights of 2,000 to 8,000, possessing a ratio of Yin-Wessler/USP titers of at least 3, a Yin-Wessler titer of at least 200 µ/mg and average molecular weights of 2,000 to 3,000.

33. GAGs according to claim 31, characterized in that they are prepared from starting materials consisting of mucopolysaccharides such as those obtained by careful depolymerisation with nitrous acid, and are composed of a mixture of chains having a terminal 2,5-anhydromanno residue, including a 2,5-anhydromannitol residue, possessing a ratio of Yin-Wassler/USP titers of 3 to 6 and average molecular weights of 4,000 to 5,000.

34. GAGs according to claim 31, characterized in that the carbon atom in position 2 of the glucosamine moiety is substituted by an $NH_2$ or NH-acyl group, including NH-acetyl.

35. GAGs according to claim 19, characterized in that they contain more than 30 sugar residues.

36. GAGs according to claim 35, characterized in that they are naturally occurring hypersulfated products, with the exception of heparin.

37. GAGs according to claim 36, characterized in that the carbon atom in position 2 of the glucosamine moiety is substituted by an $NH_2$ or NH-acyl group, in particular NH-acetyl.

38. Active principle of a medicine, characterized in that it is constituted of at least one GAG with modified sulfation pattern according to claim 19.

39. Pharmaceutical composition, characterized in that it contains an efficacious amount of at least one GAG according to any one of the claims 19 or 37, in combination with a pharmaceutical vehicle.

40. Composition according to claim 39, characterized in that it is presented in the form of a sterile, injectable solution containing from 1 to 200 mg/ml of GAGs, preferably from 20 to 150 mg/ml, when these solutions are intended for subcutaneous injection, or from 30 to 100 mg/ml particularly from 40 to 50 mg/ml of GAGs when they are intended for intravenous injection or perfusion.

41. Composition according to claim 39, characterized in that is presented in a form permitting oral administration such as gastrointestinal capsules, tablets or lozenges, pills or liposomes.

42. Composition according to claim 39, characterized in that it is presented in the form of a suppository.

43. Biological reagent, characterized in that it consists of a GAG with a modified sulfation profile according to any one of the claims 19 to 37.

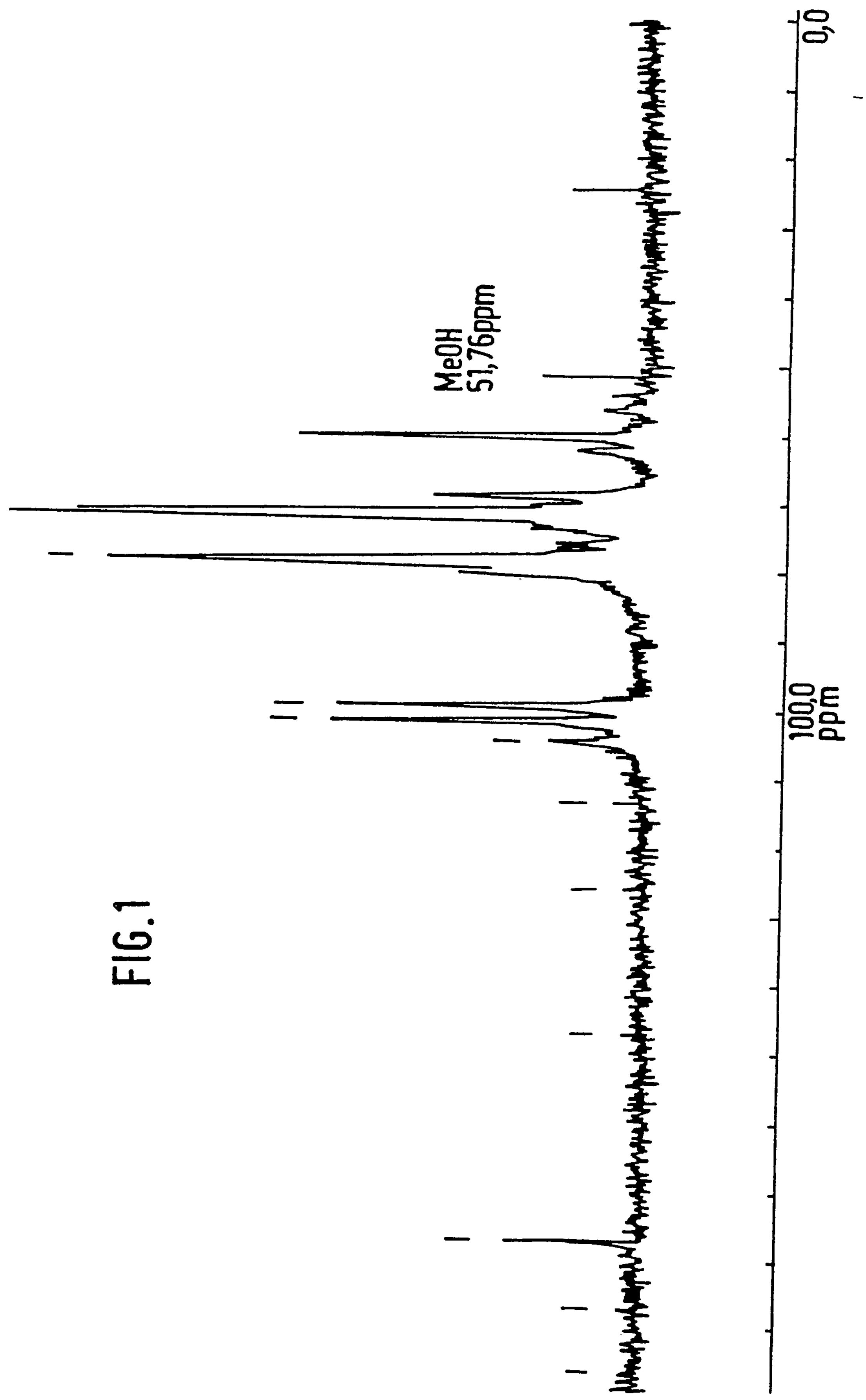


FIG.1

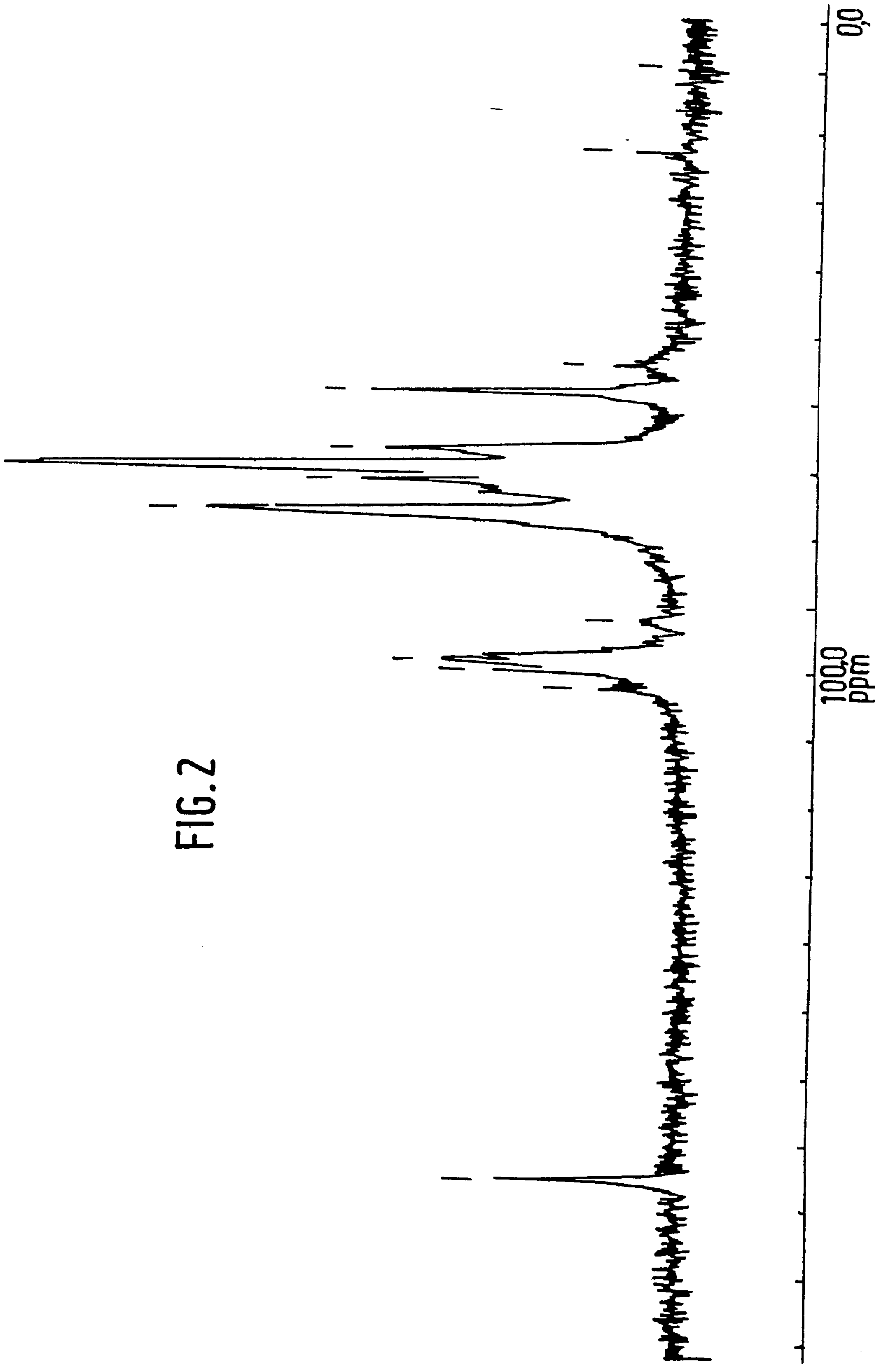


FIG. 2

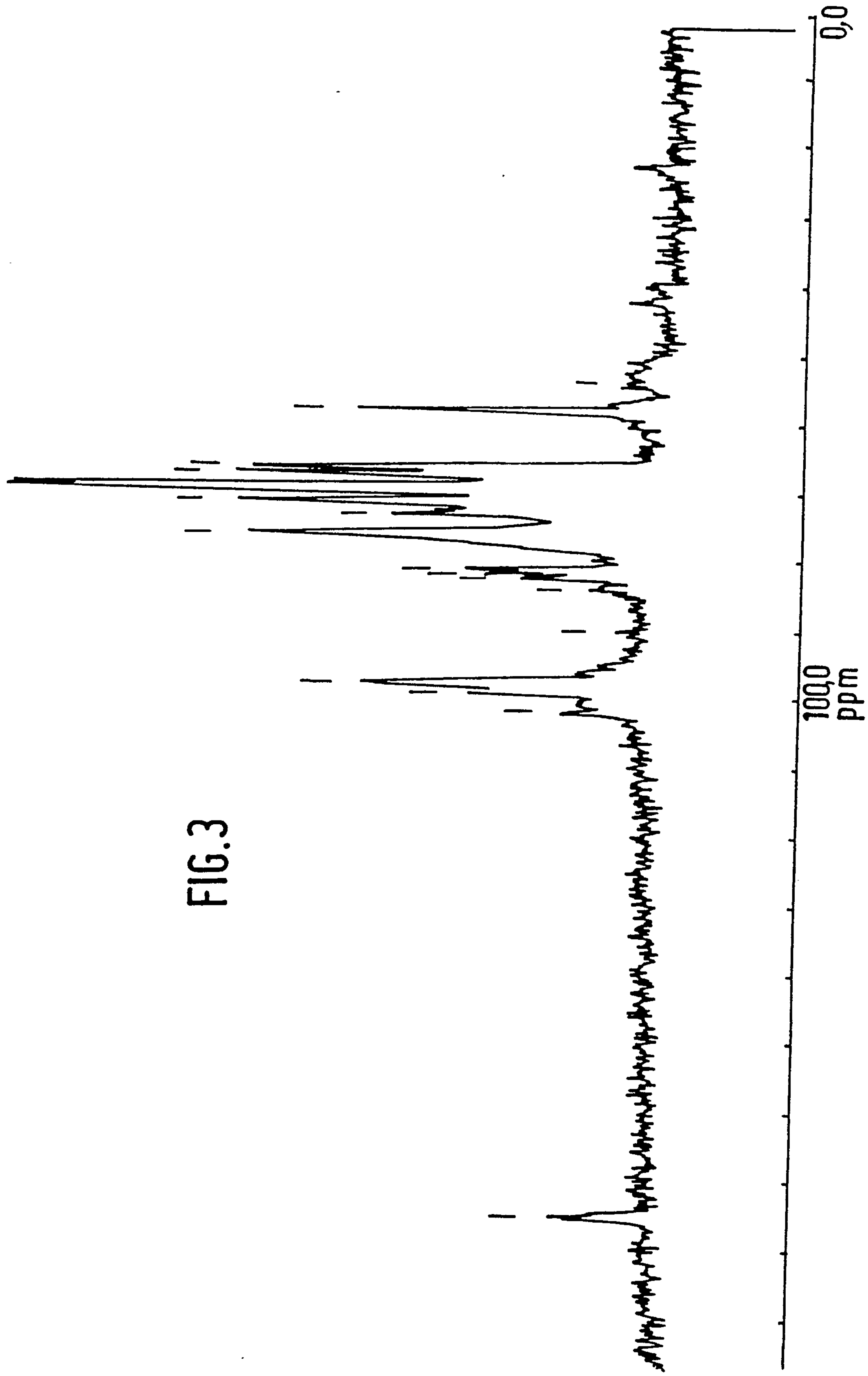


FIG.3

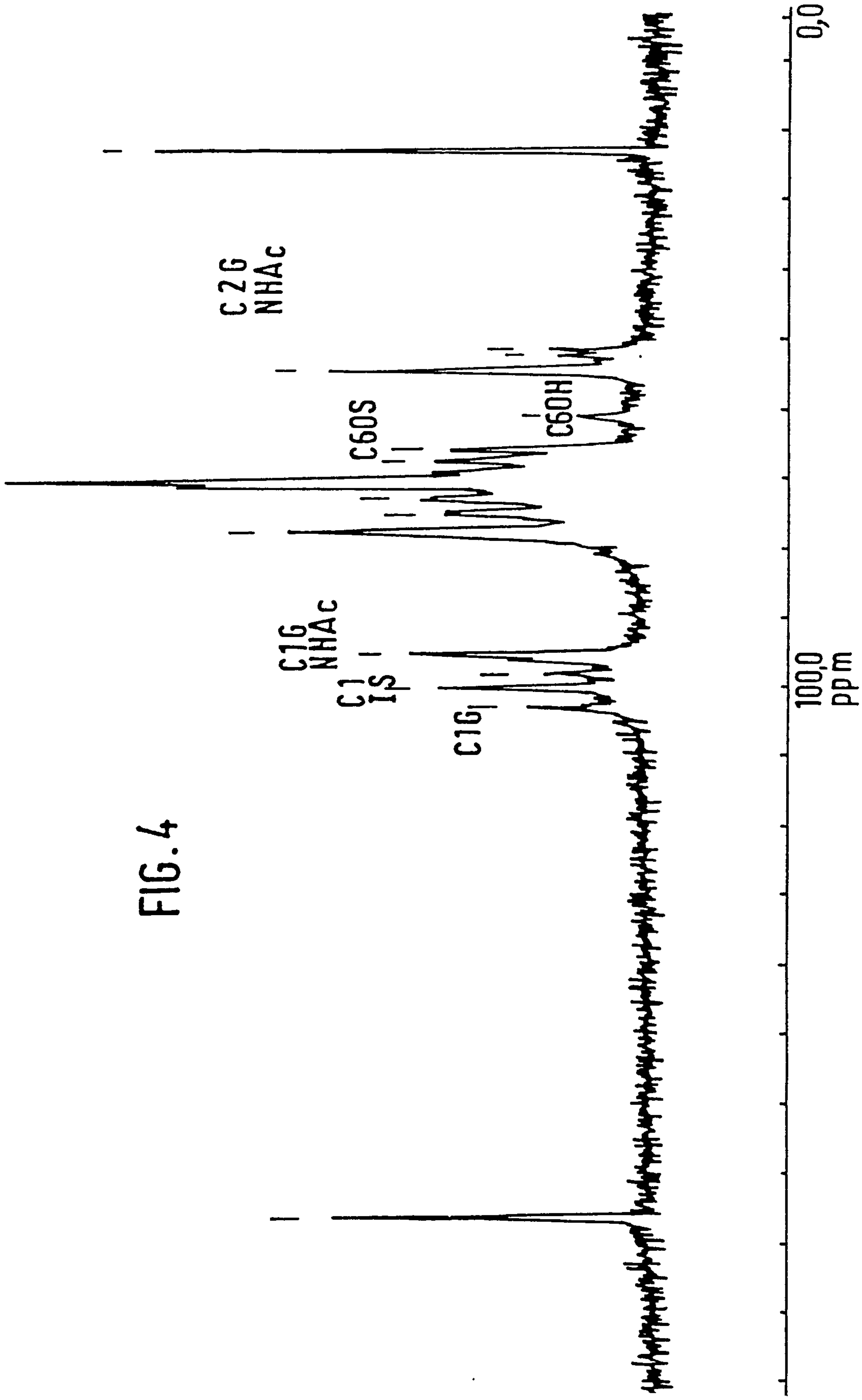


FIG. 4

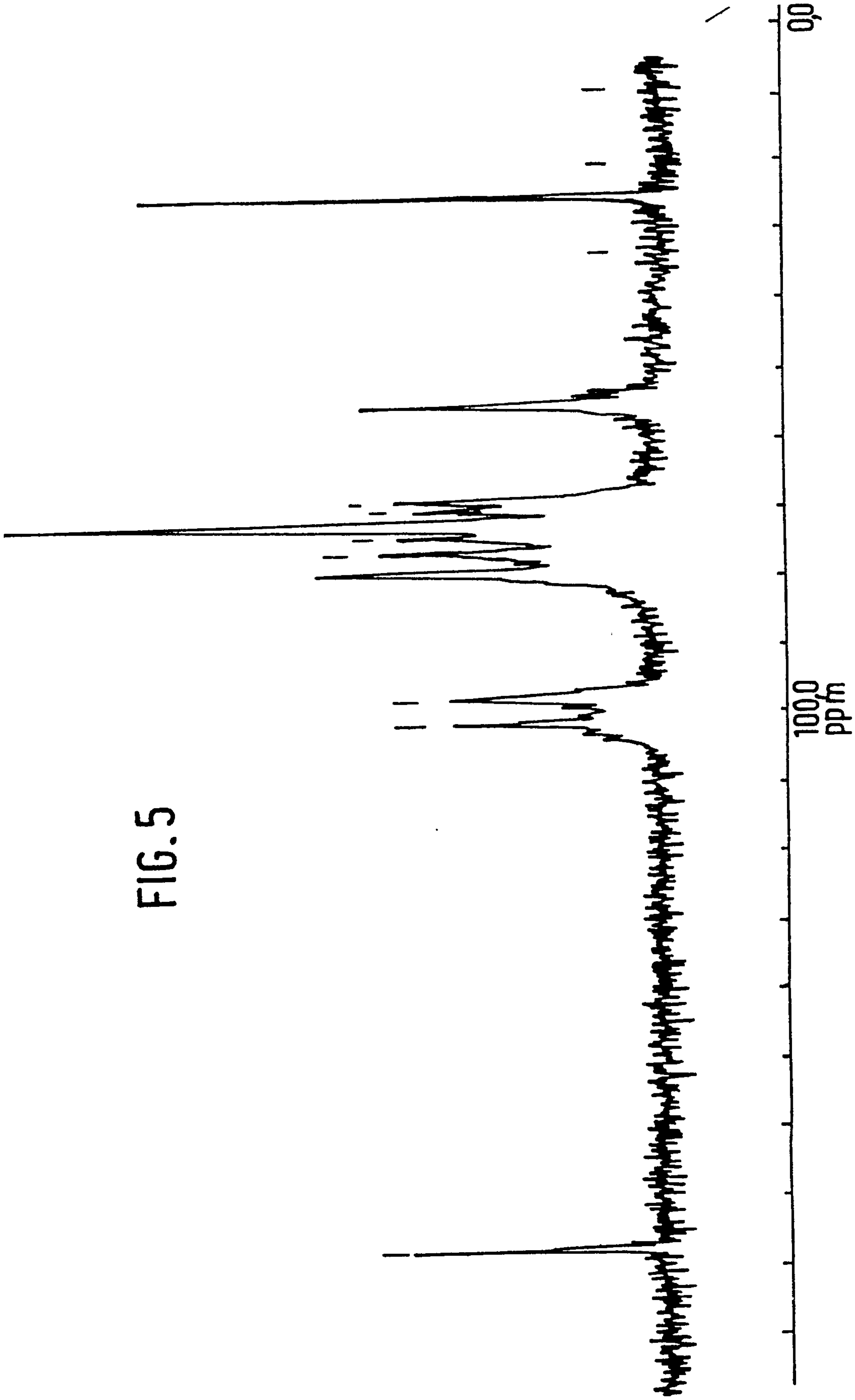


FIG. 5